# EUROPÄISCHE PATENTANMELDUNG

(11) **EP 2 229 813 A1**
(43) Veröffentlichungstag der Anmeldung: **22.09.2010**
(21) Anmeldenummer: 09004084.1
(22) Anmeldetag: 21.03.2009
(51) Int. Cl.: A01N 43/54, C07D 239/30, C07D 239/42, A01P 13/00

(54) **Pyrimidin-4-ylpropandinitril-derivate, Verfahren zu deren Herstellung sowie deren Verwendung als Herbizide und Pflanzenwachstumsregulatoren**

(71) Anmelder: Bayer CropScience AG, 40789 Monheim (DE)
(72) Erfinder: Brünjes, Marco, Dr., 65719 Hofheim (DE); Dietrich, Hansjörg, Dr., 65835 Liederbach (DE); Ahrens, Hartmut, Dr., 63329 Egelsbach (DE); Hoffmann, Michael, Gerhard, Dr., 65439 Flörsheim (DE); Dittgen, Jan, Dr., 60316 Frankfurt (DE); Feucht, Dieter, Dr., 65760 Eschborn (DE); Rosinger, Christopher, Dr., 65719 Hofheim (DE); Häuser-Hahn, Isolde, Dr., 51375 Leverkusen (DE)

(57) **Zusammenfassung**

Die Erfindung betrifft Verbindungen der Formel (I) und deren N-Oxide, Tautomere und agrochemisch verträglichen Salze

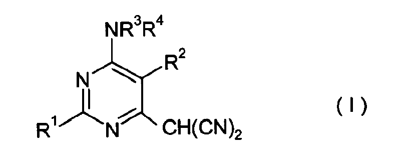

Verfahren zu deren Herstellung, sowie deren Verwendung als Herbizide und Pflanzenwachstumsregulatoren, insbesondere als Herbizide zur selektiven Bekämpfung von Schadpflanzen in Nutzpflanzenkulturen.

## Beschreibung

Die vorliegende Erfindung betrifft neue, herbizid wirksame Pyrimidin-4-ylpropandinitril-Derivate sowie Verfahren zu deren Herstellung. Weiterer Gegenstand der vorliegenden Erfindung ist deren Verwendung als Herbizid, insbesondere als Herbizid zur selektiven Bekämpfung von Schadpflanzen in Nutzpflanzenkulturen, und als Pflanzenwachstumsregulator allein oder in Kombination mit Safenern und/oder in Mischung mit anderen Herbiziden.

Aus verschiedenen Schriften ist bekannt, dass substituierte Pyrimidin-Derivate herbizide bzw. schädlingsbekämpfende Eigenschaften besitzen (siehe beispielsweise WO 2005/063721, WO 2007/082076, sowie C. Lamberth, Heterocycles 2006, 68, 3, 561-603 und dort zitierte Literatur). Die aus den oben genannten Schriften bekannten Wirkstoffe weisen bei ihrer Anwendung jedoch Nachteile auf, z. B. dass sie (a) keine oder aber eine nur unzureichende herbizide Wirkung gegen Schadpflanzen, (b) ein zu geringes Spektrum der bekämpften Schadpflanzen, oder (c) eine zu geringe Selektivität in Nutzpflanzenkulturen besitzen.

Es ist deshalb wünschenswert, chemische Wirkstoffe bereitzustellen, die mit Vorteilen als Herbizide oder Pflanzenwachstumsregulatoren eingesetzt werden können.

Es wurde nun überraschend gefunden, dass bestimmte substituierte Pyrimidin-4-ylpropandinitril-Derivate gute herbizide Wirkung und gleichzeitig hohe Verträglichkeit gegenüber Nutzpflanzen aufweisen. Ein Gegenstand der vorliegenden Erfindung sind daher Verbindungen der Formel (I) und deren N-Oxide und agrochemisch geeignete Salze, 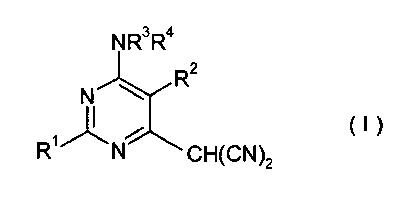 worin die Reste die folgende Bedeutung aufweisen:
R¹ ist Phenyl, optional substituiert mit 1-3 Resten R^{x}, welche unabhängig
   voneinander bedeuten Halogen, Cyano, Nitro, (C₁-C₆)Alkyl, (C₁-C₆)Haloalkyl, (C₁-C₄)Hydroxyalkyl, (C₂-C₄)Alkoxyalkyl, (C₂-C₄)Haloalkoxyalkyl, (C₂-C₄)Alkenyl, (C₂-C₄)Haloalkenyl, (C₂-C₄)Alkinyl, (C₃-C₄)Haloalkinyl, Hydroxy, (C₁-C₄)Alkoxy, (C₁-C₄)Haloalkoxy, (C₂-C₄)Alkenyloxy, (C₂-C₄)Haloalkenyloxy, (C₂-C₄)Alkinyloxy, (C₃-C₄)Haloalkinyloxy, (C₁-C₄)Alkylthio, (C₁-C₄)Haloalkylthlo, (C₁-C₄)Alkylsulfinyl, (C₁-C₄)Haloalkylsulfinyl, (C₁-C₄)Alkylsulfonyl, (C₁-C₄)Haloalkylsulfonyl, (C₂-C₄)Alkenylthio, (C₂-C₄)Haloalkenylthio, (C₂-C₄)Alkenylsulfinyl, (C₂-C₄)Haloalkenylsulfinyl, (C₂-C₄)Alkenylsulfonyl, (C₂-C₄)Haloalkenylsulfonyl, (C₂-C₄)Alkinylthio, (C₃-C₄)Haloalkinylthio, (C₃-C₄)Alkinylsulfinyl, (C₃-C₄)Haloalkinylsulfinyl, (C₃-C₄)Alkinylsulfonyl, (C₃-C₄)Haloalkinylsulfonyl, (C₁-C₆)Alkylamino, (C₂-C₈)Dialkylamino, (C₂-C₆)Alkylcarbonyl, (C₂-C₆)Alkoxycarbonyl, (C₂-C₆)Alkylaminocarbonyl, (C₃-C₈)Dialkylaminocarbonyl, (C₃-C₆)Trialkylsilyl, Phenyl, Phenoxy oder einen 5- oder 6-gliedrigen heteroaromatischen Ring, jeder Phenyl-Ring, Phenoxy-Ring oder 5-oder 6-gliedrige heteroaromatische Ring optional substituiert mit 1-3 Substituenten unabhängig voneinander ausgewählt aus R²⁸; oder zwei benachbarte Reste R^{x} bilden gemeinsam eine Gruppe
   -OCH₂O-, -CH₂CH₂O-, -OCH₂CH₂O-, -OCH(CH₃)O-, -OC(CH₃)₂O-, -OCF₂O-, -CF₂CF₂O-, -OCF₂CF₂O- oder -CH=CH-CH=CH-; oder
   (C₁-C₆)-Alkyl, optional substituiert mit 1-3 Resten R^{y}, welche unabhängig voneinander Halogen, (C₂-C₆)Alkenyl, (C₂-C₆)Haloalkenyl, (C₁-C₃)Alkoxy, (C₁-C₂)Haloalkoxy, (C₁-C₃)Alkylthio oder (C₁-C₂)Haloalkylthio bedeuten; oder
   (C₂-C₆)-Alkenyl, optional substituiert mit 1-3 Resten R^{z}, welche unabhängig voneinander Halogen, (C₁-C₆)Alkyl, (C₁-C₆)Haloalkyl, (C₁-C₃)Alkoxy, (C₁- C₂)Haloalkoxy, (C₁-C₃)Alkylthio oder (C₁-C₂)Haloalkylthio bedeuten;
   R² ist H, F, Cl, Br, I, CN, NO₂, OR⁵, SR⁶ oder N(R⁷)R⁸; worin R⁵ H, (C₁-C₄)Alkyl oder (C₁-C₃)Haloalkyl ist, R⁶ H, (C₁-C₄)Alkyl oder (C₁-C₃)Haloalkyl ist und R⁷ und R⁸ sind unabhängig H oder (C₁-C₄)Alkyl;
   R³ ist H, (C₁-C₄)Alkyl optional substituiert mit 1-2 Resten R⁹, (C₂-C₄)Alkenyl optional substituiert mit 1-2 Resten R¹⁰, oder (C₂-C₄)Alkinyl optional substituiert
   mit 1-2 Resten R¹¹; oder R³ ist C(=O)R¹², NO₂, OR¹³, S(O)₂R¹⁴, N(R¹⁵)R¹⁶ oder
   N=C(R¹⁷)R¹⁸;
   R⁴ ist H, (C₁-C₄)Alkyl optional substituiert mit Resten 1-2 R⁹, oder C(=O)R¹²; oder
   R³ und R⁴ bilden gemeinsam eine Gruppe -(CH₂)₄-, -(CH₂)₅-, -CH₂CH=CHCH₂- oder
   -(CH₂)₂O(CH₂)₂-, jede Gruppe optional substituiert mit 1-2 Resten R¹⁹; oder
   R³ und R⁴ bilden gemeinsam eine Gruppe =C(R²⁰)N(R²¹)R²² oder =C(R²³)OR²⁴;
   dabei ist jeder Rest R⁹, R¹⁰ und R¹¹ unabhängig voneinander Halogen, (C₁-
   C₃)Alkoxy, (C₁-C₃)Haloalkoxy, (C₁-C₃)Alkylthio, (C₁-C₃)Haloalkylthio, Amino, (C₁-C₃)Alkylamino, (C₂-C₄)Dialkylamino oder (C₂-C₄)Alkoxycarbonyl;
   R¹² ist unabhängig voneinander H, (C₁-C₄)Alkyl, (C₁-C₃)Haloalkyl, (C₁-C₄)Alkoxy, Phenyl, Phenoxy oder Benzyloxy;
   R¹³ ist H, (C₁-C₄)Alkyl, (C₁-C₃)Haloalkyl oder CHR²⁵C(O)OR²⁶;
   R¹⁴ ist (C₁-C₄)Alkyl oder (C₁-C₃)Haloalkyl;
   R¹⁵ ist H, (C₁-C₄)Alkyl oder C(=O)R²⁷;
   R¹⁶ ist H oder (C₁-C₄)Alkyl;
   R¹⁷ ist H, (C₁-C₄)Alkyl oder Phenyl optional substituiert mit 1-3 Resten, welche voneinander unabhängig CH₃, Cl oder OCH₃ bedeuten;
   R¹⁸ ist H oder (C₁-C₄)Alkyl; oder R¹⁷ und R¹⁸ bilden gemeinsam eine Gruppe -(CH₂)₄- oder -(CH₂)₅-;
   R¹⁹ ist unabhängig voneinander Halogen, (C₁-C₃)Alkyl, (C₁-C₃)Alkoxy, (C₁-C₃)Haloalkoxy, (C₁-C₃)Alkylthio, (C₁-C₃)Haloalkylthio, Amino, (C₁-C₃)Alkylamino, (C₂-C₄)Dialkylamino oder (C₂-C₄)Alkoxycarbonyl;
   R²⁰ ist H oder (C₁-C₄)Alkyl;
   R²¹ und R²² sind unabhängig voneinander H oder (C₁-C₄)Alkyl; oder R²¹ und R²² bilden gemeinsam eine Gruppe -(CH₂)₄-, -(CH₂)₅-, -CH₂CH=CHCH₂- oder
   -(CH₂)₂O(CH₂)₂-;

   R²³ ist H oder (C₁-C₄)Alkyl;
   R²⁴ ist (C₁-C₄)Alkyl;
   R²⁵ ist H, (C₁-C₄)Alkyl, (C₁-C₃)Haloalkyl, (C₁-C₄)Alkoxy, Phenyl, Phenoxy oder Benzyloxy;
   R²⁶ ist H, (C₁-C₄)Alkyl oder (C₁-C₄)Alkoxy;
   R²⁷ ist H, C₁-C₄ Alkyl oder Benzyl; und
   R²⁸ ist unabhängig voneinander Halogen, Cyano, Nitro, (C₁-C₆)Alkyl, (C₁-C₆)Haloalkyl, (C₂-C₄)Alkenyl, (C₂-C₄)Haloalkenyl, (C₃-C₄)Alkinyl, (C₃-C₄)Haloalkinyl, (C₁-C₄)Alkoxy, (C₁-C₄)Haloalkoxy, (C₁-C₄)Alkylthio, (C₁-C₄)Haloalkylthio, (C₁-C₄)Alkylsulfinyl, (C₁-C₄)Alkylsulfonyl, (C₁-C₄)Alkylamino, (C₂-C₈)Dialkylamino, (C₂-C₄)Alkylcarbonyl, (C₂-C₆)Alkoxycarbonyl, (C₂-C₆)Alkylaminocarbonyl, (C₃-C₈)Dialkylaminocarbonyl oder (C₃-C₆)Trialkylsilyl.

Dabei ist bei den genannten Resten stets die Gesamtzahl an C-Atomen angegeben, z.B. ist ein Dimethylaminocarbonyl-Rest ein C₃-Rest.

Bevorzugt sind Verbindungen der Formel (I), worin die Reste folgende Bedeutung aufweisen:
R¹ ist Phenyl, optional substituiert mit 1-3 Resten R^{x}, welche unabhängig
   voneinander bedeuten Halogen, Cyano, Nitro, (C₁-C₆)Alkyl, (C₁-C₆)Haloalkyl, (C₂-C₄)Alkoxyalkyl, (C₂-C₄)Haloalkoxyalkyl, (C₂-C₄)Alkenyl, (C₂-C₄)Haloalkenyl, (C₂-C₄)Alkinyl, (C₃-C₄)Haloalkinyl, Hydroxy, (C₁-C₄)Alkoxy, (C₁-C₄)Haloalkoxy, (C₁-C₄)Alkylthio, (C₁-C₄)Haloalkylthio, (C₁-C₄)Alkylsulfinyl, (C₁-C₄)Alkylsulfonyl, (C₁-C₆)Alkylamino, (C₂-C₈)Dialkylamino, -OCH₂O-, -CH₂CH₂O-, -OCH₂CH₂O-, -OCH(CH₃)O-, -OC(CH₃)₂O-, -OCF₂O-, -CF₂CF₂O- oder -OCF₂CF₂O-; oder
   (C₁-C₆)Alkyl, optional substituiert mit 1-3 Resten R^{y}, welche unabhängig voneinander bedeuten Halogen, (C₂-C₆)Alkenyl, (C₂-C₆)Haloalkenyl, (C₁-C₃)Alkoxy, (C₁-C₂)Haloalkoxy, (C₁-C₃)Alkylthio oder (C₁-C₂)Haloalkylthio; oder
   (C₂-C₆)Alkenyl, optional substituiert mit 1-3 Resten R^{z}, welche unabhängig voneinander bedeuten Halogen, (C₁-C₆)Alkyl, (C₁-C₆)Haloalkyl, (C₁-C₃)Alkoxy, (C₁-C₂)Haloalkoxy, (C₁-C₃)Alkylthio oder (C₁-C₂)Haloalkylthio;
   R² ist H, F, Cl, Br, I, CN oder NO₂;
   R³ ist H, (C₁-C₄)Alkyl, (C₂-C₄)Alkenyl, (C₂-C₄)Alkinyl, C(=O)R¹², OR¹³, N(R¹⁵)R¹⁶ oder N=C(R¹⁷)R¹⁸;
   R⁴ ist H oder (C₁-C₄)Alkyl, optional substituiert mit 1-2 Resten R⁹, oder C(=O)R¹²; oder
   R³ und R⁴ bilden gemeinsam eine Gruppe -(CH₂)₄-, -(CH₂)₅-, -(CH₂)₂O(CH₂)₂- oder
   =C(R²⁰)N(R²¹)R²²;
   dabei ist jeder Rest R⁹, R¹⁰ und R¹¹ unabhängig voneinander Halogen, (C₁-C₃)Alkoxy, (C₁-C₃)Haloalkoxy, (C₁-C₃)Alkylthio, (C₁-C₃)Haloalkylthio, Amino, (C₁-C₃)Alkylamino, (C₂-C₄)Dialkylamino oder (C₂-C₄)Alkoxycarbonyl;
   R¹² ist H, (C₁-C₄)Alkyl, (C₁-C₃)Haloalkyl, (C₁-C₄)Alkoxy, Phenyl, Phenoxy oder Benzyloxy;
   R¹³ ist H, (C₁-C₄)Alkyl, (C₁-C₃)Haloalkyl oder CHR²⁵C(O)OR²⁶;
   R¹⁵ ist H, (C₁-C₄)Alkyl oder C(=O)R²⁷;
   R¹⁶ ist H oder (C₁-C₄)Alkyl;
   R¹⁷ ist H, (C₁-C₄)Alkyl oder Phenyl optional substituiert mit 1-3 Resten, welche voneinander unabhängig CH₃, Cl oder OCH₃ bedeuten;
   R¹⁸ ist H oder (C₁-C₄)Alkyl; oder R¹⁷ und R¹⁸ bilden gemeinsam eine Gruppe -(CH₂)₄- oder -(CH₂)₅-;
   R²⁰ ist H oder (C₁-C₄)Alkyl;
   R²¹ und R²² sind unabhängig voneinander H oder (C₁-C₄)Alkyl; oder R²¹ and R²²
   bilden gemeinsam eine Gruppe -(CH₂)₄-, -(CH₂)₅-, -CH₂CH=CHCH₂- oder
   -(CH₂)₂O(CH₂)₂-; R²⁵ ist H, (C₁-C₄)Alkyl, (C₁-C₃)Haloalkyl, (C₁-C₄)Alkoxy, Phenyl, Phenoxy oder Benzyloxy; und
   R²⁶ ist H, (C₁-C₄)Alkyl oder (C₁-C₄)Alkoxy.

Besonders bevorzugt sind Verbindungen der Formel (I), worin die Reste folgende Bedeutung aufweisen:
R¹ ist Phenyl, optional substituiert mit 1-3 Resten R^{x}, welche unabhängig voneinander bedeuten Halogen, Cyano, Nitro, (C₁-C₆)Alkyl, (C₁-C₆)Haloalkyl, (C₁-C₄)Alkoxy, (C₁-C₄)Haloalkoxy, (C₁-C₆)Alkylamino, (C₂-C₈)Dialkylamino,
   -OCH₂O-, -OCH₂CH₂O- oder -OCH(CH₃)O-; oder (C₁-C₆)Alkyl, optional substituiert mit 1-3 Resten R^{y}, welche unabhängig voneinander bedeuten Halogen, (C₁-C₃)Alkoxy oder (C₁-C₂)Haloalkoxy; oder
   (C₂-C₆)Alkenyl, optional substituiert mit 1-3 Resten R^{z}, welche unabhängig voneinander bedeuten Halogen, (C₁-C₆)Alkyl, (C₁-C₆)Haloalkyl, (C₁-C₃)Alkoxy oder (C₁-C₂)Haloalkoxy;
   R² ist F, Cl, Br oder I;
   R³ ist H, (C₁-C₄)Alkyl, (C₂-C₄)Alkenyl, C(=O)R¹², worin R¹² H, (C₁-C₄)Alkyl, (C₁-C₃)Haloalkyl, (C₁-C₄)Alkoxy, Phenyl, Phenoxy oder Benzyloxy ist, oder OR¹³, worin R¹³ H, (C₁-C₄)Alkyl, (C₁-C₃)Haloalkyl oder CHR²⁵C(O)OR²⁶ ist, worin R²⁵ H, (C₁-C₄)Alkyl, (C₁-C₃)Haloalkyl, (C₁-C₄)Alkoxy, Phenyl, Phenoxy oder Benzyloxy ist, und R²⁶ H, (C₁-C₄)Alkyl oder (C₁-C₄)Alkoxy ist;
   R⁴ ist H oder (C₁-C₄)Alkyl; oder
   R³ und R⁴ bilden gemeinsam eine Gruppe -(CH₂)₂O(CH₂)₂-.

Die Verbindungen der Formel (I) können durch Wasserstoffverschiebung Tautomere bilden, welche ebenfalls Gegenstand der vorliegenden Erfindung sind. So können beispielsweise die Pyrimidin-4-ylpropandinitril-Derivate (I) auch als Pyrimidin-4(3*H*)-ylidenpropandinitrile (la) oder als Pyrimidin-4(*1H*)-ylidenpropandinitrile (Ib) vorliegen, wobei erfindungsgemäß sämtliche Tautomere umfasst sind, insbesondere die Formen (I), (Ia) und (Ib).

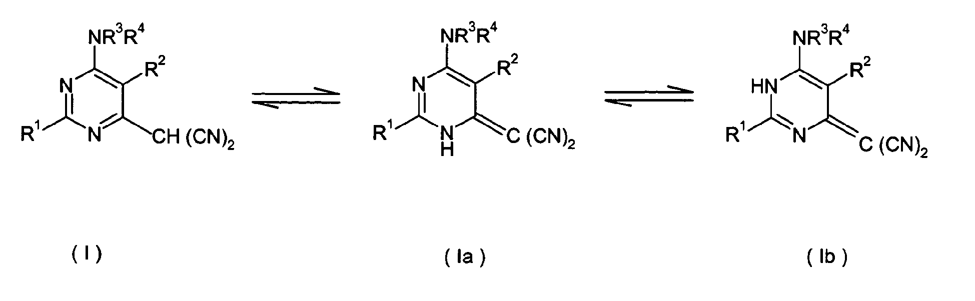

Die Verbindungen der Formel (I) können je nach Art und Verknüpfung der Substituenten als Stereoisomere vorliegen. Die durch ihre spezifische Raumform definierten möglichen Stereoisomeren, wie Enantiomere, Diastereomere, Z- und E-Isomere, geometrische Isomere und Atropisomere und deren Gemische sind alle von Formel (I) umfasst.

Sind beispielsweise eine oder mehrere Alkenylgruppen vorhanden, so können Diastereomere (Z- und E-Isomere) auftreten. Sind beispielsweise ein oder mehrere asymmetrische Kohlenstoffatome ( = asymmetrisch substituierte Kohlenstoffatome) vorhanden und/oder asymmetrische Schwefelatome in Form von Sulfoxiden, die in zwei enantiomeren Formen existieren können, so können Enantiomere und Diastereomere auftreten. Stereoisomere lassen sich aus den bei der Herstellung anfallenden Gemischen nach üblichen Trennmethoden, beispielsweise durch chromatographische Trennverfahren, isolieren. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft somit auch alle Stereoisomeren, die von der allgemeinen Formel (I) umfasst werden, auch wenn sie nicht mit ihrer spezifischen Stereoform angegeben sind, und deren Gemische.

Die Verbindungen der Formel (I) können agrochemisch geeignete Salze bilden. Salzbildung kann in bekannter Weise, z. B. durch Einwirkung einer Base auf solche Verbindungen der Formel (I) erfolgen, die ein acides Wasserstoffatom tragen, z.B. im Falle dass R¹ eine OH-Gruppe oder eine Sulfonamid-Gruppe -NHSO₂- enthält. Eine Salzbildung kann ebenfalls durch das Einwirken einer Base auf CH-acide Verbindungen, wie (substituierte) Propandinitrile, erfolgen. Geeignete Basen sind beispielsweise organische Amine, wie Trialkylamine, Morpholin, Piperidin oder Pyridin sowie Ammoniak, Ammonium-, Alkali- oder Erdalkalimetallhydroxide, -carbonate und -hydrogencarbonate, insbesondere Natrium- und Kaliumhydroxid, Natrium- und Kaliumcarbonat und Natrium- und Kaliumhydrogencarbonat. Diese Salze sind Verbindungen, in denen der acide Wasserstoff durch ein für die Landwirtschaft geeignetes Kation ersetzt wird, beispielsweise Metallsalze, insbesondere Alkalimetallsalze oder Erdalkalimetallsalze, insbesondere Natrium- und Kaliumsalze, oder auch Salze mit organischen Aminen oder Ammoniumsalze, zum Beispiel mit Ammoniumionen der Formel [NRR'R"R"']⁺, worin R, R', R" und R"' jeweils unabhängig voneinander H oder einen organischen Rest, insbesondere (C₁-C₆)Alkyl, (C₆-C₁₀)Aryl, (C₇-C₂₀)Aralkyl oder (C₇-C₂₀)Alkylaryl darstellen. Beispiele sind [NH₄]⁺, [NH₃CH₃]⁺, [NH₂(CH₃)₂]⁺, [NH(CH₃)₃]⁺, [N(CH₃)₄]⁺, [NH₂CH₃C₂H₅]⁺ oder [NH₂CH₃C₆H₅]⁺. In Frage kommen auch Alkylsulfonium- und Alkylsulfoxoniumsalze, wie (C₁-C₄)-Trialkylsulfonium- und (C₁-C₄)-Trialkylsulfoxoniumsalze.

Die Verbindungen der Formel (I) können durch Anlagerung einer geeigneten anorganischen oder organischen Säure, beispielsweise Mineralsäuren, wie beispielsweise HCl, HBr, H₂SO₄, H₃PO₄ oder HNO₃, oder organische Säuren, z. B. Carbonsäuren, wie Ameisensäure, Essigsäure, Propionsäure, Oxalsäure, Milchsäure oder Salicylsäure, oder Sulfonsäuren, wie zum Beispiel p-Toluolsulfonsäure, an eine basische Gruppe, wie Amino, Alkylamino, Dialkylamino, Piperidino, Morpholino oder Pyridino, Salze bilden. Diese Salze enthalten dann die konjugierte Base der Säure als Anion. Geeignete Substituenten, die in deprotonierter Form, wie z.B. Sulfonsäuren, Carbonsäuren oder CH-acide Gruppen, wie Propandinitrile, vorliegen, können auch innere Salze mit ihrerseits protonierbaren Gruppen, wie Aminogruppen, bilden.

Die Verbindungen der Formel (I) können N-Oxide bilden. Die N-Oxide können in bekannter Weise z. B. durch Oxidation der jeweiligen Pyrimidine mit Peroxycarbonsäuren oder Wasserstoffperoxid in Lösungsmitteln wie Acetonitril, Dichlormethan, Chloroform, Aceton, DMF, Essigsäure, z. B. bei Temperaturen zwischen 0° und 100°C gebildet werden (siehe: S. von Angerer, Science of Synthesis 2003, 16, 548-550 und die jeweils darin zitierte Literatur).

Die Verbindungen der Formel (I) und deren N-Oxide und agrochemisch geeigneten Salze werden auch kurz als erfindungsgemäß verwendete oder erfindungsgemäße Verbindungen bezeichnet. Die vorstehend und weiter unten verwendeten Bezeichnungen sind dem Fachmann geläufig und haben insbesondere die im Folgenden erläuterten Bedeutungen:
Ein anorganischer Rest ist ein Rest ohne Kohlenstoffatome, vorzugsweise Halogen, OH und dessen anorganische Salze, bei denen das H durch ein Kation, beispielsweise Alkalimetall- und Erdalkalimetallsalze ersetzt wird, -NH₂ und dessen Ammoniumsalze mit (anorganischen) Säuren, beispielsweise Mineralsäuren, -N₃ (Azid), -N₂⁺A⁻ (Diazonium-Gruppe, wobei A- ein Anion darstellt), -NO, -NHOH, -NHNH₂, -NO₂, -ONO, -ONO₂, -SH, -SOH (Sulfensäure-Gruppe), -S(O)OH (Sulfinsäure-Gruppe), -S(O)₂OH (oder auch kurz SO₃H, Sulfonsäure-Gruppe), -O-SO₂H (Sulfit-Gruppe), -O-SO₃H (Sulfat-Gruppe), -SO₂NH₂ (Sulfamoyl-Gruppe), -SO₂NHOH (Hydroxysulfamoyl-Gruppe), -NHS(O)OH (Sulfinoamino-Gruppe), -NHS(O)₂OH (Sulfoamino-Gruppe), -P(O)(OH)₂ (Phosphonsäure-Gruppe), -O-P(OH)₃ (Phosphat-Gruppe), -P(O)(NH₂)₂, -PO(OH)(NH₂), -PS(OH)₂, -PS(NH₂)₂ oder -PS(OH)(NH₂), -B(OH)₂ (Boronsäure-Gruppe) und die hydratisierten oder dehydratisierten Formen der Säuregruppen sowie deren (anorganischen) Salze;
der Begriff "anorganischer Rest" umfasst auch den Wasserstoffrest (das Wasserstoffatom), wobei dieser in den Definitionen oft bereits Bestandteil des unsubstituierten Grundkörpers eines organischen Restes ist (Beispiel "unsubstituiertes Phenyl");
der Begriff "anorganischer Rest" umfasst hier vorzugsweise nicht Pseudohalogen-Gruppen wie CN, SCN, organische Metallkomplexe, Carbonat oder COOH, die wegen des Gehalts an C-Atomen den organischen Resten zugeordnet werden.

Die Bezeichnung "Halogen" oder "Halogenatom" bedeutet beispielsweise Fluor, Chlor, Brom oder Iod.

Wird die Bezeichnung für einen Rest verwendet, dann bedeutet "Halogen" oder "Halogenatom" beispielsweise ein Fluor-, Chlor-, Brom- oder Iodatom.

Alkyl bedeutet einen geradkettigen, verzweigten oder cyclischen Kohlenwasserstoffrest. Der Ausdruck "(C₁-C₄)Alkyl" bedeutet beispielsweise eine Kurzschreibweise für Alkyl mit einem bis 4 Kohlenstoffatomen entsprechend der Bereichsangabe für C-Atome und umfasst z.B. die Reste Methyl, Ethyl, 1-Propyl, 2-Propyl, 1-Butyl, 2-Butyl, 2-Methylpropyl, tert-Butyl, Cyclopropyl und Cyclobutyl. Allgemeine Alkylreste mit einem größeren angegebenen Bereich von C-Atomen, z. B. "(C₁-C₆)Alkyl", umfassen entsprechend auch gradkettige, verzweigte oder cyclische Alkylreste mit einer größeren Zahl von C-Atomen, d. h. gemäß Beispiel auch die Alkylreste mit 5 und 6 C-Atomen.

Wenn nicht speziell angegeben, sind bei den Kohlenwasserstoffresten wie Alkyl-, Alkenyl- und Alkinylresten, auch in zusammengesetzten Resten, die niederen Kohlenstoffgerüste, z.B. mit 1 bis 6 C-Atomen bzw. bei ungesättigten Gruppen mit 2 bis 6 C-Atomen, bevorzugt. Alkylreste, auch in den zusammengesetzten Resten wie Alkoxy, Haloalkyl usw., bedeuten z.B. Methyl, Ethyl, Cyclo-, n- oder i-Propyl, Cyclo-, n-, i-, t- oder 2-Butyl, Pentyle, Hexyle, wie Cyclohexyl, n-Hexyl, i-Hexyl und 1,3-Dimethylbutyl, Heptyle, wie Cycloheptyl, n-Heptyl, 1-Methylhexyl und 1,4-Dimethylpentyl.

Bevorzugte cyclische Alkylreste weisen vorzugsweise 3-8 Ring-C-Atome auf, z.B. Cyclopropyl, Cyclobutyl, Cyclopentyl oder Cyclohexyl. Im Falle von gegebenenfalls substituierten cyclischen Alkylresten werden cyclische Systeme mit Substituenten umfasst, wobei auch Substituenten mit einer Doppelbindung am cyclischen Alkylrest, z. B. eine Alkylidengruppe wie Methyliden, umfasst sind.

Im Falle von gegebenenfalls substituierten cyclischen Alkylresten werden auch mehrcyclische aliphatische Systeme umfaßt, wie Bicyclo[1.1.0]butan-1-yl, Bicyclo[1.1.0]butan-2-yl, Bicyclo[2.1.0]pentan-1-yl, Bicyclo[2.1.0]pentan-2-yl, Bicyclo[2.1.0]pentan-5-yl, Bicyclo[2.2.1]hept-2-yl (Norbornyl), Adamantan-1-yl und Adamantan-2-yl.

Im Falle von gegebenenfalls substituierten cyclischen Alkylresten werden auch spirocyclische aliphatische Systeme umfaßt, wie beispielsweise Spiro[2.2]pent-1-yl, Spiro[2.3]hex-1-yl, Spiro[2.3]hex-4-yl, 3-Spiro[2.3]hex-5-yl.

Alkenyl- und Alkinylreste haben die Bedeutung der den Alkylresten entsprechenden möglichen ungesättigten geradkettigen, verzweigten oder cyclischen Reste, wobei mindestens eine Doppelbindung bzw. Dreifachbindung enthalten ist. Bevorzugt sind Reste mit einer Doppelbindung bzw. mit einer Dreifachbindung.

Alkenyl schließt auch geradkettige, verzweigte oder cyclische Kohlenwasserstoffreste mit mehr als einer Doppelbindung ein, wie 1,3-Butadienyl, 1,4-Pentadienyl oder Cyclohexadienyl, aber auch Allenyl- oder Kumulenyl-reste mit einer bzw. mehreren kumulierten Doppelbindungen, wie beispielsweise Allenyl (1,2-Propadienyl), 1,2-Butadienyl und 1,2,3-Pentatrienyl.

Alkinyl schließt auch geradkettige, verzweigte oder cyclische Kohlenwasserstoffreste mit mehr als einer Dreifachbindung oder auch mit einer oder mehreren Dreifachbindungen und einer oder mehreren Doppelbindungen ein, wie beispielsweise 1,3-Butatrienyl bzw. 3-Penten-1-in-1-yl.

Alkenyl bedeutet z.B. Vinyl, welches ggf. durch weitere Alkylreste substituiert sein kann, z.B. Prop-1-en-1-yl, But-1-en-1-yl;
Allyl, 1-Methyl-prop-2-en-1-yl, 2-Methyl-prop-2-en-1-yl, But-2-en-1-yl, 1-Methyl-but-3-en-1-yl und 1-Methyl-but-2-en-1-yl, 2-Methylprop-1-en-1-yl, 1-Methylprop-1-en-1-yl, 1-Methylprop-2-en-1-yl, 2-Methyl-prop-2-en-1-yl, But-2-en-1-yl, But-3-en-1-yl, 1-Methyl-but-3-en-1-yl oder 1-Methyl-but-2-en-1-yl, Pentenyl, 2-Methylpentenyl oder Hexenyl.

(C₂-C₆) -Alkinyl bedeutet beispielsweise Ethinyl, Propargyl, 1-Methyl-prop-2-in-1-yl, 2-Butinyl, 2-Pentinyl oder 2-Hexinyl, vorzugsweise Propargyl, But-2-in-1-yl, But-3-in-1-yl oder 1-Methyl-but-3-in-1-yl.

Cyclische Alkenylreste bedeuten ein carbocyclisches, nicht aromatisches, partiell ungesättigtes Ringsystem mit vorzugsweise 4-8 C-Atomen, z.B. 1-Cyclobutenyl, 2-Cyclobutenyl, 1-Cyclopentenyl, 2-Cyclopentenyl, 3-Cyclopentenyl, oder 1-Cyclohexenyl, 2-Cyclohexenyl, 3-Cyclohexenyl, 1,3-Cyclohexadienyl oder 1,4-Cyclohexadienyl, wobei auch Substituenten mit einer Doppelbindung am Cycloalkenylrest, z. B. eine Alkylidengruppe wie Methyliden, umfasst sind. Im Falle von gegebenenfalls substituiertem Cycloalkenyl gelten die Erläuterungen für substituierte cyclische Alkylreste entsprechend.

Alkyliden, z. B. auch in der Form (C₁-C₁₀)Alkyliden, bedeutet den Rest eines geradkettigen, verzweigten oder cyclischen Kohlenwasserstoffrests, der über eine Zweifachbindung gebunden ist. Als Bindungsstelle für Alkyliden kommen naturgemäß nur Positionen am Grundkörper in Frage, an denen zwei H-Atome durch die Doppelbindung ersetzt werden können; Reste sind z. B. =CH₂, =CH-CH₃, =C(CH₃)-CH₃, =C(CH₃)-C₂H₅ oder =C(C₂H₅)-C₂H₅.

Aryl bedeutet ein mono-, bi- oder polycyclisches aromatisches System mit vorzugsweise 6 bis 14, insbesondere 6 bis 10 Ring-C-Atomen, beispielsweise Phenyl, Naphthyl, Anthryl, Phenanthrenyl, und ähnliches, vorzugsweise Phenyl.

Im Falle gegebenenfalls substituierten Aryls sind auch mehrcyclische Systeme, wie Tetrahydronaphtyl, Indenyl, Indanyl, Fluorenyl, Biphenylyl, umfasst, wobei die Bindungsstelle am aromatischen System ist. Von der Systematik her ist Aryl in der Regel auch von dem Begriff "gegebenenfalls substituiertes Phenyl" umfasst.

Mit der Definition "mit einem oder mehreren Resten substituiert ist" sind, wenn nicht anders definiert, unabhängig voneinander ein oder mehrere gleiche oder verschiedene Reste gemeint, wobei zwei oder mehrere Reste an einem Cyclus als Grundkörper einen oder mehrere Ringe bilden können.

Substituierte Reste, wie ein substituierter Alkyl-, Alkenyl-, Alkinyl-, Aryl-, Phenyl-, Benzyl-, Heterocyclyl- und Heteroarylrest, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Alkylthio, Hydroxy, Amino, Nitro, Carboxy oder eine der Carboxygruppe äquivalente Gruppe, Cyano, Isocyano, Azido, Alkoxycarbonyl, Alkylcarbonyl, Formyl, Carbamoyl, Mono- und Dialkylaminocarbonyl, substituiertes Amino, wie Acylamino, Mono- und Dialkylamino, Trialkylsilyl und gegebenenfalls substituiertes cyclisches Alkyl, gegebenenfalls substituiertes Aryl, gegebenenfalls substituiertes Heterocyclyl, wobei jeder der letztgenannten cyclischen Gruppen auch über Heteroatome oder divalente funktionelle Gruppen wie bei den genannten Alkylresten gebunden sein kann, und Alkylsulfinyl, wobei beide Enantiomere der Alkylsulfonylgruppe umfasst sind, Alkylsulfonyl Alkylphosphinyl, Alkylphosphonyl und, im Falle cyclischer Reste (= "cyclischer Grundkörper"), auch Alkyl, Haloalkyl, Alkylthio-alkyl, Alkoxy-alkyl, gegebenfalls substituiertes Mono- und Dialkyl-aminoalkyl und Hydroxy-alkyl bedeuten;

im Begriff "substituierte Reste" wie substituiertes Alkyl (z.B. gradkettiges, verzweigtes oder cyclisches Alkyl) etc. sind als Substituenten zusätzlich zu den genannten gesättigten kohlenwasserstoffhaltigen Resten entsprechende ungesättigte aliphatische und aromatische Reste, wie gegebenenfalls substituiertes Alkenyl, Alkinyl, Alkenyloxy, Alkinyloxy, Alkenylthio, Alkinylthio, Alkenyloxycarbonyl, Alkinyloxycarbonyl, Alkenylcarbonyl, Alkinylcarbonyl, Mono- und Dialkenylaminocarbonyl, Mono- und Dialkinylaminocarbonyl, Mono- und Dialkenylamino, Mono- und Dialkinylamino, Trialkenylsilyl, Trialkinylsilyl, Phenyl, Phenoxy etc. eingeschlossen. Im Falle von substituierten cyclischen Resten mit aliphatischen Anteilen im Ring werden auch cyclische Systeme mit solchen Substituenten umfaßt, die mit einer Doppelbindung am Ring gebunden sind, z. B. mit einer Alkylidengruppe wie Methyliden oder Ethyliden oder einer Oxogruppe, Iminogruppe oder substituierten Iminogruppe substituiert sind.

Wenn zwei oder mehrere Reste einen oder mehrere Ringe bilden, so können diese carbocyclisch, heterocyclisch, gesättigt, teilgesättigt, ungesättigt, beispielsweise auch aromatisch und gegebenenfalls weiter substituiert sein. Die annellierten Ringe sind vorzugsweise 5- oder 6-Ringe, besonders bevorzugt sind benzokondensierte Cyclen.

Die beispielhaft genannten Substituenten ("erste Substituentenebene") können, sofern sie kohlenwasserstoffhaltige Anteile enthalten, dort gegebenenfalls weiter substituiert sein ("zweite Substitutentenebene"), beispielsweise durch einen der Substituenten, wie er für die erste Substituentenebene definiert ist. Entsprechende weitere Substituentenebenen sind möglich. Vorzugsweise werden vom Begriff "substituierter Rest" nur ein oder zwei Substitutentenebenen umfasst.

Bevorzugte Substituenten für die Substituentenebenen sind beispielsweise Amino, Hydroxy, Halogen, Nitro, Cyano, Isocyano, Mercapto, Isothiocyanato, Carboxy, Carbonamid, SF₅, Aminosulfonyl, Alkyl, Alkenyl, Alkinyl, Monoalkyl-amino, Dialkyl-amino, N-Alkanoyl-amino, Alkoxy, Alkenyloxy, Alkinyloxy, Alkoxy-carbonyl, Alkenyloxy-carbonyl, Alkinyloxy-carbonyl, Aryloxycarbonyl, Alkanoyl, Alkenylcarbonyl, Alkinyl-carbonyl, Aryl-carbonyl, Alkylthio, Alkenylthio, Alkinylthio, Alkylsulfenyl, Alkylsulfinyl, wobei beide Enantiomere der Alkylsulfinylgruppe umfasst sind, Alkylsulfonyl, Monoalkyl-aminosulfonyl, Dialkyl-aminosulfonyl, Alkylphosphinyl, Alkylphosphonyl, wobei für Alkylphosphinyl bzw. Alkylphosphonyl beide Enantiomere umfasst sind, N-Alkyl-aminocarbonyl, N,N-Dialkyl-aminocarbonyl, N-Alkanoyl-aminocarbonyl, N-Alkanoyl-N-alkyl-aminocarbonyl, Aryl, Aryloxy, Benzyl, Benzyloxy, Benzylthio, Arylthio, Arylamino, Benzylamino, Heterocyclyl und Trialkylsilyl.

Substituenten, die aus mehreren Substituentenebenen zusammengesetzt sind, sind bevorzugt beispielsweise Alkoxyalkyl, wie Monoalkoxyalkyl oder Dialkoxyalkyl, Alkylthioalkyl, Alkylthioalkoxy, Alkoxyalkoxy, wie Monoalkoxyalkoxy oder Dialkoxyalkoxy, Benzyl, Phenethyl, Benzyloxy, Haloalkyl, Haloalkoxy, Haloalkylthio, Haloalkanoyl, Haloalkylcarbonyl, Haloalkoxycarbonyl, Haloalkoxyalkoxy, Haloalkoxyalkylthio, Haloalkoxyalkanoyl, Haloalkoxyalkyl, Alkanoylalkyl, Haloalkanoylalkyl, Alkanoyloxyalkyl.

Bei Resten mit C-Atomen sind solche mit 1 bis 6 C-Atomen, vorzugsweise 1 bis 4 C-Atomen, insbesondere 1 oder 2 C-Atomen bevorzugt. Bevorzugt sind in der Regel Substituenten aus der Gruppe Halogen, z.B. Fluor und Chlor, (C₁-C₄)Alkyl, vorzugsweise Methyl oder Ethyl, (C₁-C₄)Haloalkyl, vorzugsweise Trifluormethyl, (C₁-C₄)Alkoxy, vorzugsweise Methoxy oder Ethoxy, (C₁-C₄)Haloalkoxy, Nitro und Cyano. Besonders bevorzugt sind dabei die Substituenten Methyl, Methoxy, Fluor und Chlor.

Substituiertes Amino wie mono- oder disubstituiertes Amino bedeutet einen Rest aus der Gruppe der substituierten Aminoreste, welche beispielsweise durch einen bzw. zwei gleiche oder verschiedene Reste aus der Gruppe Alkyl, Hydroxy, Amino, Alkoxy, Acyl und Aryl N-substituiert sind; vorzugsweise Mono- und Dialkyl-amino, Mono- und Diarylamino, wie gegebenenfalls substituierte Aniline, Acylamino, N,N-diacylamino, N-Alkyl-N-arylamino, N-Alkyl-N-acylamino sowie gesättigte N-Heterocyclen; dabei sind Alkylreste mit 1 bis 4 C-Atomen bevorzugt; Aryl ist dabei vorzugsweise Phenyl oder substituiertes Phenyl; für Acyl gilt dabei die weiter unten genannte Definition, vorzugsweise (C₁-C₄)Alkanoyl. Entsprechenes gilt für substituiertes Hydroxylamino oder Hydrazino.

Substituiertes Amino schließt auch quartäre Ammoniumverbindungen (Salze) mit vier organischen Substituenten am Stickstoffatom ein.

Eine der Carboxygruppe äquivalente Gruppe bedeutet beispielsweise ein Alkylester, Arylester, O-Alkylthioester, S-Alkyldithioester, S-Alkylthioester, Carboximidester, Carboximidthioester; 5,6-Dihydro-1,2,4-dioxazin-3-yl; 5,6-Dihydro-1,2,4-oxathiazin-3-yl, Trialkylorthoester, Dialkoxyalkylaminoester, Dialkylaminoalkoxyester, Trialkylaminoester, Amidine, Dialkoxyketenacetale oder Dialkyldithioketenacetale.

Gegebenenfalls substituiertes Phenyl ist vorzugsweise Phenyl, das unsubstituiert oder ein- oder mehrfach, vorzugsweise bis zu dreifach durch gleiche oder verschiedene Reste aus der Gruppe Halogen, (C₁-C₄)Alkyl, (C₁-C₄)Alkoxy, (C₁-C₄)Alkoxy-(C₁-C₄)alkoxy, (C₁-C₄)Alkoxy-(C₁-C₄)alkyl, (C₁-C₄)Halogenalkyl, (C₁-C₄)Halogenalkoxy, (C₁-C₄)Alkylthlo, (C₁-C₄)Halogenalkylthio, Cyano, Isocyano und Nitro substituiert ist, z.B. o-, m- und p-Tolyl, Dimethylphenyle, 2-, 3- und 4-Chlorphenyl, 2-, 3- und 4-Fluorphenyl, 2-, 3- und 4-Trifluormethyl- und -Trichlormethylphenyl, 2,4-, 3,5-, 2,5- und 2,3-Dichlorphenyl, o-, m- und p-Methoxyphenyl.

Gegebenenfalls substituiertes Heterocyclyl ist vorzugsweise Heterocyclyl, das unsubstituiert oder ein- oder mehrfach, vorzugsweise bis zu dreifach durch gleiche oder verschiedene Reste aus der Gruppe Halogen, Cyano, (C₁-C₄)Alkyl, (C₁-C₄)Alkoxy, (C₁-C₄)Alkoxy-(C₁-C₄)alkoxy, (C₁-C₄)Alkoxy-(C₁-C₄)alkyl, (C₁-C₄)Halogenalkyl, (C₁-C₄)Halogenalkoxy, Nitro und Oxo substituiert ist, insbesondere ein- oder mehrfach durch Reste aus der Gruppe Halogen, (C₁-C₄)Alkyl, (C₁-C₄)Alkoxy, (C₁-C₄)Halogenalkyl und Oxo, ganz besonders durch einen oder zwei (C₁-C₄)Alkylreste substituiert ist.

Haloalkyl, -alkenyl und -alkinyl bedeuten durch gleiche oder verschiedene Halogenatome, teilweise oder vollständig substituiertes Alkyl, Alkenyl bzw. Alkinyl, z.B. Monohaloalkyl (= Monohalogenalkyl) wie CH₂CH₂Cl, CH₂CH₂F, CHCICH₃, CHFCH₃, CH₂Cl, CH₂F; Perhaloalkyl wie CCl₃ oder CF₃ oder CF₂CF₃; Polyhaloalkyl wie CHF₂, CH₂F, CH₂CHFCl, CHCl₂, CF₂CF₂H, CH₂CF₃; Haloalkoxy ist z.B. OCF₃, OCHF₂, OCH₂F, OCF₂CF₃, OCH₂CF₃ und OCH₂CH₂Cl; Entsprechendes gilt für Haloalkenyl und andere durch Halogen substituierten Reste.

Ein organischer Säurerest bedeutet einen Rest einer Oxosäure oder Thiosäure der allgemeinen Formel

R*ₖ*E(=Q)*ₚ*(OH)(QR')*ₙ*

wobei
R ein organischer Rest,
E ein Atom aus der Gruppe C, S, P,
Q unabhängig voneinander ein Atom oder ein Molekülfragment aus der Gruppe O, S, NR' und
R' unabhängig voneinander ein Wasserstoffatom, Alkyl, Haloalkyl, Alkoxyalkyl oder gegebenenfalls Aryl bedeutet.
*k, p* sind natürliche Zahlen, *k* = 1, 2; *p* = 0 - 2;
*n* ist eine natürliche Zahl oder Null.

Der organische Säurerest entsteht formal durch Abtrennen einer Hydroxygruppe an der Säurefunktion, wobei der organische Rest R in der Säure auch über ein oder mehrere Heteroatome mit der Säurefunktion verbunden sein kann:

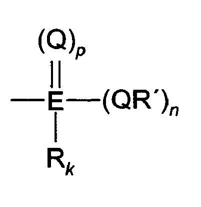

Für Oxosäuren des Kohlenstoffs ist dies im IUPAC Compendium of Chemical Terminology (1997) beschrieben.

Beispiele für organische Säurereste, die von den Oxosäuren bzw. Thiosäuren des Schwefels abgeleitet sind (E = S), sind S(O)OCH₃, SO₂OH, SO₂OCH₃ oder SO₂NHR (N-substituierte Sulfonamidsäuren).

Im Falle von *k* = 1 sind auch Alkylsulfonyl- und Alkylsulfinyl-Reste, wie z.B.
(H₃C)S(O)₂, (F₃C)S(O)₂, p-TolylS(O)₂, (H₃C)S(O)(NH-n-C₄H₉), (C₆H₅)S(S)(O) oder (C₆H₅)S(O) mit umfasst.

Beispiele für organische Säurereste, die von den Oxosäuren bzw. Thiosäuren des Phosphors abgeleitet sind (E = P), sind von der Phosphinsäure und der Phosphonsäure abgeleitete Reste, wobei diese Reste weiter verestert sein können, z.B. -PO(OCH₃)₂, (C₂H₅O)P(O)OH_{,} (C₂H₅O)P(O)(SC₆H₅), (H₃CO)P(O)NH(C₆H₅) oder -PO(NMe₂)₂.

Im Falle von *k* = 1 sind auch Alkylphosphinyl- und Alkylphosphonyl-Reste, wie z.B.
(H₃C)₂ P(O), (C₆H₅)₂P(O), (H₃C)(C₆H₅)P(O); (H₃C)P(O)OCH₃, (H₅C₂)P(O)(OC₂H₅), (C₆H₅)P(O)(OC₂H₅), (C₂H₅)P(O)(SC₆H₅), (H₃C)P(O)NH(C₆H₅), (H₃C)P(S)(NH-i-C₃H₇), (C₆H₅)P(S)(OC₂H₅) oder (C₆H₅)P(S)(SC₂H₅) mit umfasst.

Organische Säurereste, die von den Oxosäuren des Kohlenstoffs abgeleitet sind (E = C, Q = O), werden im engeren Sinne auch mit dem Begriff "Acyl" bezeichnet.

Beispiele für Acyl sind der Rest -CO-R einer Carbonsäure HO-CO-R und Reste davon abgeleiteter Säuren oder der Rest von Kohlensäuremonoestern oder N-substituierter Carbaminsäuren sowie Carbonate und deren Ester.

Acyl bedeutet beispielsweise Formyl, Oxalyl(ester), Alkylcarbonyl wie [(C₁-C₄)Alkyl]-carbonyl, Haloalkylcarbonyl, Phenylcarbonyl, Alkyloxycarbonyl, speziell tert.-Butyloxycarbonyl, Phenyloxycarbonyl, Benzyloxycarbonyl, Fluorenyloxycarbonyl, N-Alkyl-1-iminoalkyl, N-Alkyl- und N,N-Dialkylcarbamoyl. Dabei können die Reste jeweils im Alkyl- oder Phenylteil noch weiter substituiert sein, beispielsweise im Alkylteil durch ein oder mehrere Reste aus der Gruppe Halogen, Cyano, Alkoxy, Phenyl und Phenoxy; Beispiele für Substituenten im Phenylteil sind die bereits weiter oben allgemein für substituiertes Phenyl erwähnten Substituenten.

Acyl bedeutet vorzugsweise einen Acylrest im engeren Sinne, d.h. einen Rest einer organischen Säure, bei der die Säuregruppe direkt mit dem C-Atom eines organischen Restes verbunden ist, beispielsweise Alkanoyl, wie Formyl und Acetyl, Aroyl wie Phenylcarbonyl, und andere Reste von gesättigten oder ungesättigten organischen Säuren.

"Aroyl" bedeutet einen wie vorstehend definierter Arylrest, der über eine CarbonylGruppe gebunden ist, z.B. die Benzoyl-Gruppe.

Wenn ein allgemeiner Rest mit "Wasserstoff" definiert ist, bedeutet dies ein Wasserstoffatom.

Mit "yl-Position" eines Restes ist dessen Bindungstelle bezeichnet.

Gegenstand der vorliegenden Erfindung sind auch Methoden zur Herstellung der erfindungsgemäßen Verbindungen. Die erfindungsgemäßen Verbindungen können alternativ durch verschiedene Verfahren dargestellt werden.

In den nachfolgenden Verfahren werden partiell Lösemittel verwendet. In diesem Zusammenhang bezeichnen "inerte Lösemittel" jeweils Lösemittel, die unter den jeweiligen Reaktionsbedingungen inert sind, jedoch nicht unter beliebigen Reaktionsbedingen inert sein müssen.

Verbindungen der Formel (I) können z. B. durch Reaktion der entsprechenden Halogenverbindungen (II) mit dem jeweiligen Amin der Formel (III) hergestellt werden, optional unter Verwendung einer organischen oder anorganischen Base (z. B. Triethylamin, Pyridin, Kaliumcarbonat, Natriumcarbonat). Die Reaktion kann in verschiedenen Lösungsmitteln wie Methanol, Ethanol, Dioxan, THF, Dichlormethan, DMSO, DMF und Wasser durchgeführt werden. Die Reaktionstemperaturen liegen je nach verwendetem Amin im Allgemeinen zwischen 20°C und 180°C.

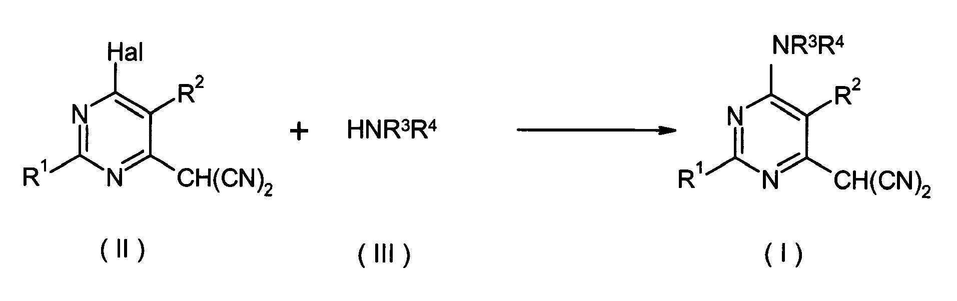

Verbindungen der Formel (II) können z. B. durch Reaktion der entsprechenden 4,6-Dihalogenverbindungen (IV) mit dem jeweiligen Alkalisalz des Malondinitrils erhalten werden. Die jeweiligen Salze können z. B. in situ durch Behandlung von Malondinitril mit verschiedenen Basen wie z.B. n-Butyllithium, Lithiumdiisopropylamid, Natriumhydrid oder Kaliumcarbonat bei Temperaturen zwischen -80°C und 80°C generiert werden. Nach Zugabe der 4,6-Dihalogenpyrimidine (IV) wird die Reaktion in einem Temperaturbereich zwischen 0°C und 100°C durchgeführt, bevorzugt in aprotischen Lösungsmitteln wie THF, DMSO, DMF oder Dioxan.

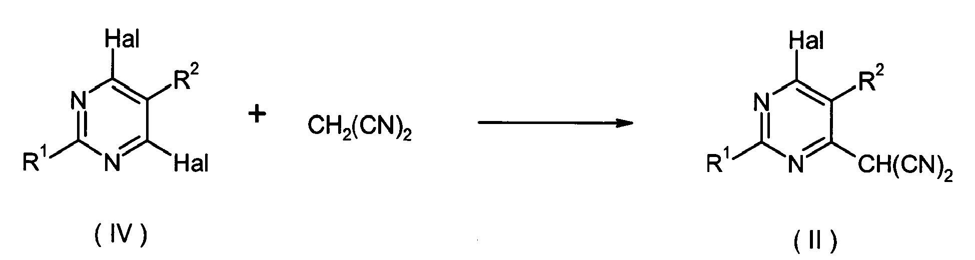

Die Herstellung von Verbindungen der Formel (IV) erfolgt durch allgemein bekannte Methoden: K. Findeisen, K. Wagner, Synthesis 1978, 40-42; H. Gershon, K. Dittmer, R. Braun, J. Org. Chem. 1961, 26, 1874-1877; H. Gershon, R. Braun, A. Scala, R. Rodin, J. Med. Chem. 1964, 7, 808-811; D. T. Hurst, Heterocycles 1984, 22, 79-84; L. Provins et al., Bioorg. Med. Chem. Lett. 2006, 16, 1834-1839; T. Sakamoto, Y. Kondo, R. Watanabe, H. Yamanaka, Chem. Pharm. Bull. 1986, 34, 2719-2724; S. von Angerer, Science of Synthesis 2003, 16, 379-572 und die jeweils darin zitierte Literatur. In den Formeln (II), (III) und (IV) haben die Reste R¹, R², R³ und R⁴ die gleiche Bedeutung wie in Formel (I), Hal in Formel (II) und (IV) bedeutet ein Halogenatom.

Kollektionen aus erfindungsgemäßen Verbindungen, die nach den oben genannten Reaktionen synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch D. Tiebes in Combinatorial Chemistry - Synthesis, Analysis, Screening (Herausgeber Günther Jung), Verlag Wiley 1999, auf den Seiten 1 bis 34 beschrieben ist.

Zur parallelisierten Reaktionsdurchführung und Aufarbeitung können eine Reihe von im Handel erhältlichen Geräten verwendet werden, beispielsweise Calpyso-Reaktionsblöcke (Caylpso reaction blocks) der Firma Barnstead International, Dubuque, lowa 52004-0797, USA oder Reaktionsstationen (reaction stations) der Firma Radleys, Shirehill, Saffron Walden, Essex, CB 11 3AZ, England oder MultiPROBE Automated Workstations der Firma Perkin Elmar, Waltham, Massachusetts 02451, USA. Für die parallelisierte Aufreinigung von erfindungsgemäßen Verbindungen beziehungsweise von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise durch Roboter bedient werden. Derartige Automationssysteme können zum Beispiel von der Firma Caliper, Hopkinton, MA 01748, USA bezogen werden.

Die Durchführung einzelner oder mehrerer Syntheseschritte kann durch den Einsatz von Polymer-supported reagents/Scavanger-Harze unterstützt werden. In der Fachliteratur sind eine Reihe von Versuchsprotokollen beschrieben, beispielsweise in ChemFiles, Vol. 4, No. 1, Polymer-Supported Scavengers and Reagents for Solution-Phase Synthesis (Sigma-Aldrich).

Neben den hier beschriebenen Methoden kann die Herstellung von erfindungsgemäßen Verbindungen vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepassten Synthese an ein Syntheseharz gebunden. Festphasen- unterstützte Synthesemethoden sind in der Fachliteratur hinreichend beschrieben, z.B. Barry A. Bunin in "The Combinatorial Index", Verlag Academic Press, 1998 und Combinatorial Chemistry - Synthesis, Analysis, Screening (Herausgeber Günther Jung), Verlag Wiley, 1999. Die Verwendung von Festphasenunterstützten Synthesemethoden erlaubt eine Reihe von literaturbekannten Protokollen, die wiederum manuell oder automatisiert ausgeführt werden können. Die Reaktionen können beispielsweise mittels IRORI-Technologie in Mikroreaktoren (microreactors) der Firma Nexus Biosystems, 12140 Community Road, Poway, CA92064, USA durchgeführt werden.

Sowohl an fester als auch in flüssiger Phase kann die Durchführung einzelner oder mehrerer Syntheseschritte durch den Einsatz der Mikrowellen-Technologie unterstützt werden. In der Fachliteratur sind eine Reihe von Versuchsprotokollen beschrieben, beispielsweise in Microwaves in Organic and Medicinal Chemistry (Herausgeber C. O. Kappe und a. Stadler), Verlag Wiley, 2005.

Die Herstellung gemäß der hier beschriebenen Verfahren liefert erfindungsgemäße Verbindungen in Form von Substanzkollektionen, die Bibliotheken genannt werden. Gegenstand der vorliegenden Erfindung sind auch Bibliotheken, die mindestens zwei erfindungsgemäße Verbindungen enthalten.

Die erfindungsgemäßen Verbindungen weisen eine ausgezeichnete herbizide Wirksamkeit gegen ein breites Spektrum wirtschaftlich wichtiger mono- und dikotyler annueller Schadpflanzen auf. Auch schwer bekämpfbare perennierende Schadpflanzen, die aus Rhizomen, Wurzelstöcken oder anderen Dauerorganen austreiben, werden durch die Wirkstoffe gut erfasst.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im Einzelnen seien beispielhaft einige Vertreter der mono- und dikotylen Unkrautflora genannt, die durch die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Schadpflanzen der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Schadpflanzen aufweisen, werden Kulturpflanzen wirtschaftlich bedeutender Kulturen wie dikotyler Kulturen, z.B. der Gattungen Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia, oder monokotyler Kulturen z.B. der Gattungen Allium, Ananas, Asparagus, Avena, Hordeum, Oryza, Panicum, Saccharum, Secale, Sorghum, Triticale, Triticum, Zea, insbesondere Zea und Triticum, abhängig von der Struktur der jeweiligen erfindungsgemäßen Verbindung und deren Aufwandmenge nur unwesentlich oder gar nicht geschädigt. Die erfindungsgemäßen Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüber hinaus weisen die erfindungsgemäßen Verbindungen (abhängig von ihrer jeweiligen Struktur und der ausgebrachten Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Des Weiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da beispielsweise die Lagerbildung hierdurch verringert oder völlig verhindert werden kann.

Aufgrund ihrer herbiziden und pflanzenwachstumsregulatorischen Eigenschaften können die erfindungsgemäßen Verbindungen auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z.B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt. Weitere besondere Eigenschaften können in einer Toleranz oder Resistenz gegen abiotische Stressoren z.B. Hitze, Kälte, Trockenheit, Salz und ultraviolette Strahlung liegen.

Bevorzugt ist die Anwendung der erfindungsgemäßen Verbindungen in wirtschaftlich bedeutenden transgenen Kulturen von Nutz-und Zierpflanzen, z.B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten.

Vorzugsweise können die erfindungsgemäßen Verbindungen als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z.B. EP 0221044, EP 0131624). Beschrieben wurden beispielsweise in mehreren Fällen
- gentechnische Veränderungen von Kulturpflanzen zwecks Modifikation der in den Pflanzen synthetisierten Stärke (z.B. WO 92/011376 A, WO 92/014827 A, WO 91/019806 A),
- transgene Kulturpflanzen, welche gegen bestimmte Herbizide vom Typ Glufosinate (vgl. z.B. EP 0 242 236 A, EP 0 242 246 A) oder Glyphosate (WO 92/000377 A) oder der Sulfonylharnstoffe (EP 0 257 993 A, US 5,013,659) oder gegen Kombinationen oder Mischungen dieser Herbizide durch "gene stacking" resistent sind, wie transgenen Kulturpflanzen z. B. Mais oder Soja mit dem Handelsnamen oder der Bezeichnung Optimum^{™} GAT^{™} (Glyphosate ALS Tolerant).
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP 0 142 924 A, EP 0 193 259 A).
- transgene Kulturpflanzen mit modifizierter Fettsäurezusammensetzung (WO 91/013972 A).
- gentechnisch veränderte Kulturpflanzen mit neuen Inhalts- oder Sekundärstoffen z.B. neuen Phytoalexinen, die eine erhöhte Krankheitsresistenz verursachen (EP 0 309 862 A, EP 0 464 461 A)
- gentechnisch veränderte Pflanzen mit reduzierter Photorespiration, die höhere Erträge und höhere Stresstoleranz aufweisen (EP 0 305 398 A)
- transgene Kulturpflanzen, die pharmazeutisch oder diagnostisch wichtige Proteine produzieren ("molecular pharming")
- transgene Kulturpflanzen, die sich durch höhere Erträge oder bessere Qualitat auszeichnen
- transgene Kulturpflanzen die sich durch eine Kombinationen z.B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt; siehe z.B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z.B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z.B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z.B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen in transgenen Kulturen eingesetzt werden, welche gegen Wuchsstoffe, wie z.B. 2,4 D, Dicamba oder gegen Herbizide, die essentielle Pflanzenenzyme, z.B. Acetolactatsynthasen (ALS), EPSP Synthasen, Glutaminsynthasen (GS) oder Hydoxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, oder gegen beliebige Kombinationen dieser Wirkstoffe, resistent sind.

Besonders bevorzugt können die erfindungsgemäßen Verbindungen in transgenen Kulturpflanzen eingesetzt werden, die gegen eine Kombination von Glyphosaten und Glufosinaten, Glyphosaten und Sulfonylharnstoffen oder Imidazolinonen resistent sind. Ganz besonders bevorzugt können die erfindungsgemäßen Verbindungen in transgenen Kulturpflanzen wie z. B. Mais oder Soja mit dem Handelsnamen oder der Bezeichnung Optimum^{™} GAT^{™} (Glyphosate ALS Tolerant) eingesetzt werden.

Bei der Anwendung der erfindungsgemäßen Verbindungen in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen als Herbizide zur Bekämpfung von Schadpflanzen in transgenen Kulturpflanzen.

Die erfindungsgemäßen Verbindungen können z.B. in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, welche die erfindungsgemäßen Verbindungen enthalten.

Die erfindungsgemäßen Verbindungen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse. Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986; Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hanser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wässrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0.1 bis 99 Gew.-%, insbesondere 0.1 bis 95 Gew.-%, erfindungsgemäße Verbindungen.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0.05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstoffformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Als Kombinationspartner für die erfindungsgemäßen Verbindungen in Mischungsformulierungen oder im Tank-Mix sind beispielsweise bekannte Wirkstoffe, wie Insektizide, Fungizide, Pflanzenwachstumsregulatoren oder Herbizide geeignet. Beispiele derartiger Herbizide sind solche, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II, Protoporphyrinogen-Oxidase beruhen, einsetzbar, wie sie z.B. aus Weed Research 26 (1986) 441 445 oder "The Pesticide Manual", 14th edition, The British Crop Protection Council and the Royal Soc. of Chemistry, 2003 und dort zitierter Literatur beschrieben sind. Als bekannte Herbizide oder Pflanzenwachstumsregulatoren, die mit den erfindungsgemäßen Verbindungen kombiniert werden können, sind z.B. folgende Wirkstoffe zu nennen (die Verbindungen sind entweder mit dem "common name" nach der International Organization for Standardization (ISO) oder mit dem chemischen Namen oder mit der Codenummer bezeichnet) und umfassen stets sämtliche Anwendungsformen wie Säuren, Salze, Ester und Isomere wie Stereoisomere und optische Isomere. Dabei sind beispielhaft eine und zum Teil auch mehrere Anwendungsformen genannt:
Acetochlor, Acibenzolar, Acibenzolar-S-methyl, Acifluorfen, Acifluorfen-natrium, Aclonifen, Alachlor, Allidochlor, Alloxydim, Alloxydim-natrium, Ametryn, Amicarbazone, Amidochlor, Amidosulfuron, Aminocyclopyrachlor, Aminopyralid, Amitrole, Ammoniumsulfamat, Ancymidol, Anilofos, Asulam, Atrazine, Azafenidin, Azimsulfuron, Aziprotryn, BAH-043, BAS-140H, BAS-693H, BAS-714H, BAS-762H, BAS-776H, Beflubutamid, Benazolin, Benazolin-ethyl, bencarbazone, Benfluralin, Benfuresate, Bensulide, Bensulfuron-methyl, Bentazone, Benzfendizone, Benzobicyclon, Benzofenap, Benzofluor, Benzoylprop, Bifenox, Bilanafos, Bilanafosnatrium, Bispyribac, Bispyribac-natrium, Bromacil, Bromobutide, Bromofenoxim, Bromoxynil, Bromuron, Buminafos, Busoxinone, Butachlor, Butafenacil, Butamifos, Butenachlor, Butralin, Butroxydim, Butylate, Cafenstrole, Carbetamide, Carfentrazone, Carfentrazone-ethyl, Chlomethoxyfen, Chloramben, Chlorazifop, Chlorazifop-butyl, Chlorbromuron, Chlorbufam, Chlorfenac, Chlorfenac-natrium, Chlorfenprop, Chlorflurenol, Chlorflurenol-methyl, Chloridazon, Chlorimuron, Chlorimuron-ethyl, Chlormequat-chlorid, Chlornitrofen, Chlorophthalim, Chlorthaldimethyl, Chlorotoluron, Chlorsulfuron, Cinidon, Cinidon-ethyl, Cinmethylin, Cinosulfuron, Clethodim, Clodinafop Clodinafop-propargyl, Clofencet, Clomazone, Clomeprop, Cloprop, Clopyralid, Cloransulam, Cloransulam-methyl, Cumyluron, Cyanamide, Cyanazine, Cyclanilide, Cycloate, Cyclosulfamuron, Cycloxydim, Cycluron, Cyhalofop, Cyhalofop-butyl, Cyperquat, Cyprazine, Cyprazole, 2,4-D, 2,4-DB, Daimuron/Dymron, Dalapon, Daminozide, Dazomet, n-Decanol, Desmedipham, Desmetryn, Detosyl-Pyrazolate (DTP), Diallate, Dicamba, Dichlobenil, Dichlorprop, Dichlorprop-P, Diclofop, Diclofop-methyl, Diclofop-P-methyl, Diclosulam, Diethatyl, Diethatyl-ethyl, Difenoxuron, Difenzoquat, Diflufenican, Diflufenzopyr, Diflufenzopyrnatrium, Dimefuron, Dikegulac-natrium, Dimefuron, Dimepiperate, Dimethachlor, Dimethametryn, Dimethenamid, Dimethenamid-P, Dimethipin, Dimetrasulfuron, Dinitramine, Dinoseb, Dinoterb, Diphenamid, Dipropetryn, Diquat, Diquat-dibromide, Dithiopyr, Diuron, DNOC, Eglinazine-ethyl, Endothal, EPTC, Esprocarb, Ethalfluralin, Ethametsulfuron-methyl, Ethephon, Ethidimuron, Ethiozin, Ethofumesate, Ethoxyfen, Ethoxyfen-ethyl, Ethoxysulfuron, Etobenzanid, F-5331, d.h. N-[2-Chlor-4-fluor-5-[4-(3-fluorpropyl)-4,5-dihydro-5-oxo-1H-tetrazol-1-yl]-phenyl]-ethansulfonamid, Fenoprop, Fenoxaprop, Fenoxaprop-P, Fenoxaprop-ethyl, Fenoxaprop-P-ethyl, Fentrazamide, Fenuron, Flamprop, Flamprop-M-isopropyl, Flamprop-M-methyl, Flazasulfuron, Florasulam, Fluazifop, Fluazifop-P, Fluazifop-butyl, Fluazifop-P-butyl, Fluazolate, Flucarbazone, Flucarbazone-natrium, Flucetosulfuron, Fluchloralin, Flufenacet (Thiafluamide), Flufenpyr, Flufenpyr-ethyl, Flumetralin, Flumetsulam, Flumiclorac, Flumiclorac-pentyl, Flumioxazin, Flumipropyn, Fluometuron, Fluorodifen, Fluoroglycofen, Fluoroglycofen-ethyl, Flupoxam, Flupropacil, Flupropanate, Flupyrsulfuron, Flupyrsulfuron-methyl-natrium, Flurenol, Flurenolbutyl, Fluridone, Flurochloridone, Fluroxypyr, Fluroxypyr-meptyl, Flurprimidol, Flurtamone, Fluthiacet, Fluthiacet-methyl, Fluthiamide, Fomesafen, Foramsulfuron, Forchlorfenuron, Fosamine, Furyloxyfen, Gibberellinsäure, Glufosinate, Glufosinateammonium, Glufosinate-P, Glufosinate-P-ammonium, Glufosinate-P-natrium, Glyphosate, Glyphosate-isopropylammonium, H-9201, Halosafen, Halosulfuron, Halosulfuron-methyl, Haloxyfop, Haloxyfop-P, Haloxyfop-ethoxyethyl, Haloxyfop-P-ethoxyethyl, Haloxyfop-methyl, Haloxyfop-P-methyl, Hexazinone, HNPC-9908, HW-02, Imazamethabenz, Imazamethabenz-methyl, Imazamox, Imazapic, Imazapyr, Imazaquin, Imazethapyr, Imazosulfuron, Inabenfide, Indanofan, Indaziflam, Indolessigsäure (IAA), 4-Indol-3-ylbuttersäure (IBA), lodosulfuron, lodosulfuronmethyl-natrium, loxynil, Ipfencarbazone, Isocarbamid, Isopropalin, Isoproturon, Isouron, Isoxaben, Isoxachlortole, Isoxaflutole, Isoxapyrifop, KUH-043, KUH-071, Karbutilate, Ketospiradox, Lactofen, Lenacil, Linuron, Maleinsäurehydrazid, MCPA, MCPB, MCPB-methyl, -ethyl und -natrium, Mecoprop, Mecoprop-natrium, Mecopropbutotyl, Mecoprop-P-butotyl, Mecoprop-P-dimethylammonium, Mecoprop-P-2-ethylhexyl, Mecoprop-P-kalium, Mefenacet, Mefluidide, Mepiquat-chlorid, Mesosulfuron, Mesosulfuron-methyl, Mesotrione, Methabenzthiazuron, Metam, Metamifop, Metamitron, Metazachlor, Methazole, Methiozolin, Methoxyphenone, Methyldymron, 1-Methylcyclopropen, Methylisothiocyanat, Metobenzuron, Metobenzuron, Metobromuron, Metolachlor, S-Metolachlor, Metosulam, Metoxuron, Metribuzin, Metsulfuron, Metsulfuron-methyl, Molinate, Monalide, Monocarbamide, Monocarbamide-dihydrogensulfat, Monolinuron, Monosulfuron, Monuron, MT 128, MT-5950, d. h. N-[3-Chlor-4-(1-methylethyl)-phenyl]-2-methylpentanamid, NGGC-011, Naproanilide, Napropamide, Naptalam, NC-310, d.h. 4-(2,4-dichlorobenzoyl)-1-methyl-5-benzyloxypyrazole, NC-620, Neburon, Nicosulfuron, Nipyraclofen, Nitralin, Nitrofen, Nitrophenolat-natrium (Isomerengemisch), Nitrofluorfen, Nonansäure, Norflurazon, Orbencarb, Orthosulfamuron, Oryzalin, Oxadiargyl, Oxadiazon, Oxasulfuron, Oxaziclomefone, Oxyfluorfen, Paclobutrazol, Paraquat, Paraquatdichlorid, Pelargonsäure (Nonansäure), Pendimethalin, Pendralin, Penoxsulam, Pentanochlor, Pentoxazone, Perfluidone, Pethoxamid, Phenisopham, Phenmedipham, Phenmedipham-ethyl, Picloram, Picolinafen, Pinoxaden, Piperophos, Pirifenop, Pirifenop-butyl, Pretilachlor, Primisulfuron, Primisulfuronmethyl, Probenazole, Profluazol, Procyazine, Prodiamine, Prifluraline, Profoxydim, Prohexadione, Prohexadione-calcium, Prohydrojasmone, Prometon, Prometryn, Propachlor, Propanil, Propaquizafop, Propazine, Propham, Propisochlor, Propoxycarbazone, Propoxycarbazone-natrium, Propyrisulfuron, Propyzamide, Prosulfalin, Prosulfocarb, Prosulfuron, Prynachlor, Pyraclonil, Pyraflufen, Pyraflufenethyl, Pyrasulfotole, Pyrazolynate (Pyrazolate), Pyrazosulfuron-ethyl, Pyrazoxyfen, Pyribambenz, Pyribambenz-isopropyl, Pyribenzoxim, Pyributicarb, Pyridafol, Pyridate, Pyriftalid, Pyriminobac, Pyriminobac-methyl, Pyrimisulfan, Pyrithiobac, Pyrithiobac-natrium, Pyroxasulfone, Pyroxsulam, Quinclorac, Quinmerac, Quinoclamine, Quizalofop, Quizalofop-ethyl, Quizalofop-P, Quizalofop-P-ethyl, Quizalofop-P-tefuryl, Rimsulfuron, Saflufenacil, Secbumeton, Sethoxydim, Siduron, Simazine, Simetryn, SN-106279, Sulcotrione, Sulfallate (CDEC), Sulfentrazone, Sulfometuron, Sulfometuron-methyl, Sulfosate (Glyphosate-trimesium), Sulfosulfuron, SYN-449, SYN-523, SYP-249, SYP-298, SYP-300, Tebutam, Tebuthiuron, Tecnazene, Tefuryltrione, Tembotrione, Tepraloxydim, Terbacil, Terbucarb, Terbuchlor, Terbumeton, Terbuthylazine, Terbutryn, Thenylchlor, Thiafluamide, Thiazafluron, Thiazopyr, Thidiazimin, Thidiazuron, Thiencarbazone, Thiencarbazone-methyl, Thifensulfuron, Thifensulfuron-methyl, Thiobencarb, Tiocarbazil, Topramezone, Tralkoxydim, Triallate, Triasulfuron, Triaziflam, Triazofenamide, Tribenuron, Tribenuron-methyl, Trichloressigsäure (TCA), Triclopyr, Tridiphane, Trietazine, Trifloxysulfuron, Trifloxysulfuron-natrium, Trifluralin, Triflusulfuron, Triflusulfuron-methyl, Trimeturon, Trinexapac, Trinexapac-ethyl, Tritosulfuron, Tsitodef, Uniconazole, Uniconazole-P, Vernolate, ZJ-0166, ZJ-0270, ZJ-0543, ZJ-0862 sowie die folgenden Verbindungen

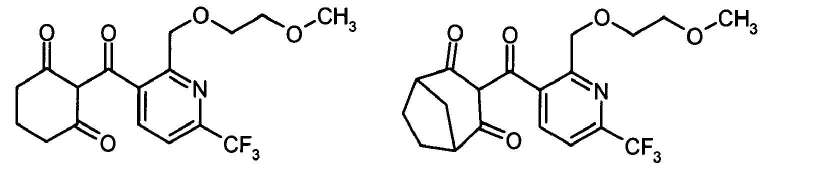

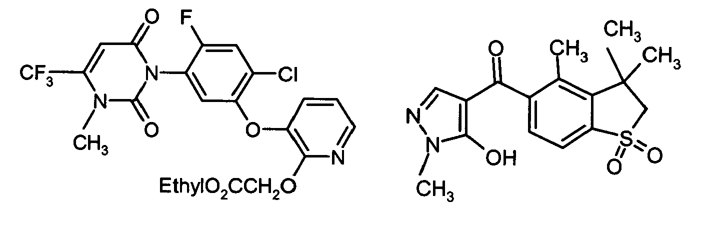

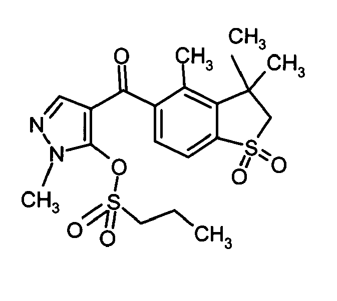

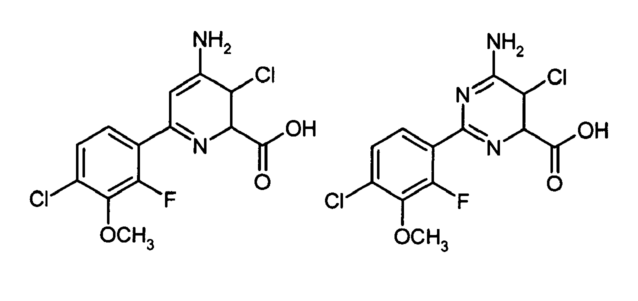

Von besonderem Interesse ist die selektive Bekämpfung von Schadpflanzen in Kulturen von Nutz- und Zierpflanzen. Obgleich die erfindungsgemäßen Verbindungen bereits in vielen Kulturen sehr gute bis ausreichende Selektivität aufweisen, können prinzipiell in einigen Kulturen und vor allem auch im Falle von Mischungen mit anderen Herbiziden, die weniger selektiv sind, Phytotoxizitäten an den Kulturpflanzen auftreten. Diesbezüglich sind Kombinationen von besonderem Interesse, welche die erfindungsgemäßen Verbindungen in Kombination mit Safenern enthalten, sowie gegebenenfalls weitere Pestizide wie Herbizide. Die Safener, welche in einem antidotisch wirksamen Gehalt eingesetzt werden, reduzieren die phytotoxischen Nebenwirkungen der eingesetzten Pestizide, z. B. in wirtschaftlich bedeutenden Kulturen wie Getreide (z.B. Weizen, Gerste, Roggen, Mais, Reis, Hirse), Zuckerrübe, Zuckerrohr, Raps, Baumwolle und Soja, vorzugsweise Getreide.

Folgende Gruppen von Verbindungen kommen beispielsweise als Safener in Frage:
S1) Verbindungen aus der Gruppe heterocyclischer Carbonsäurederivate:
   S1^{a}) Verbindungen vom Typ der Dichlorphenylpyrazolin-3-carbonsäure (S1^{a}),
      vorzugsweise Verbindungen wie 1-(2,4-Dichlorphenyl)-5-(ethoxycarbonyl)-5-methyl-2-pyrazolin-3-carbonsäure, 1-(2,4-Dichlorphenyl)-5-(ethoxycarbonyl)-5-methyl-2-pyrazolin-3-carbonsäure-ethylester (S1-1) ("Mefenpyr-diethyl"), und verwandte Verbindungen, wie sie in der WO-A-91/07874 beschrieben sind;
   S1^{b}) Derivate der Dichlorphenylpyrazolcarbonsäure (S1^{b}),
      vorzugsweise Verbindungen wie 1-(2,4-Dichlorphenyl)-5-methylpyrazol-3-carbonsäureethylester (S1-2), 1-(2,4-Dichlorphenyl)-5-isopropylpyrazol-3-carbonsäureethylester (S1-3), 1-(2,4-Dichlorphenyl)-5-(1,1-dimethyl-ethyl)pyrazol-3-carbonsäureethylester (S1-4) und verwandte Verbindungen, wie sie in EP-A-333 131 und EP-A-269 806 beschrieben sind;
   S1^{c}) Derivate der 1,5-Diphenylpyrazol-3-carbonsäure (S1^{c}),
      vorzugsweise Verbindungen wie 1-(2,4-Dichlorphenyl)-5-phenylpyrazol-3-carbonsäureethylester (S1-5), 1-(2-Chlorphenyl)-5-phenylpyrazol-3-carbonsäuremethylester (S1-6) und verwandte Verbindungen wie sie beispielsweise in der EP-A-268554 beschrieben sind;
   S1^{d}) Verbindungen vom Typ der Triazolcarbonsäuren (S1^{d}),
      vorzugsweise Verbindungen wie Fenchlorazol(-ethylester), d.h. 1-(2,4-Dichlorphenyl)-5-trichlormethyl-(1H)-1,2,4-triazol-3-carbonsäure-ethylester (S1-7), und verwandte Verbindungen, wie sie in EP-A-174 562 und EP-A-346 620 beschrieben sind;
   S1^{e}) Verbindungen vom Typ der 5-Benzyl- oder 5-Phenyl-2-isoxazolin-3- carbonsäure, oder der 5,5-Diphenyl-2-isoxazolin-3-carbonsäure (S1^{e}), vorzugsweise Verbindungen wie
      5-(2,4-Dichlorbenzyl)-2-isoxazolin-3-carbonsäureethylester (S1-8) oder
      5-Phenyl-2-isoxazolin-3-carbonsäureethylester (S1-9) und verwandte Verbindungen, wie sie in WO-A-91/08202 beschrieben sind, bzw. 5,5-Diphenyl-2-isoxazolin-carbonsäure (S1-10) oder 5,5-Diphenyl-2-isoxazolin-carbonsäureethylester (S1-11) ("Isoxadifen-ethyl") oder
      -n-propylester (S1-12) oder der 5-(4-Fluorphenyl)-5-phenyl-2-isoxazolin-3-carbonsäureethylester (S1-13), wie sie in der Patentanmeldung WO-A-95/07897 beschrieben sind.
S2) Verbindungen aus der Gruppe der 8-Chinolinyloxyderivate (S2):
   S2^{a}) Verbindungen vom Typ der 8-Chinolinoxyessigsäure (S2^{a}), vorzugsweise (5-Chlor-8-chinolinoxy)essigsäure-(1-methylhexyl)-ester ("Cloquintocetmexyl") (S2-1), (5-Chlor-8-chinolinoxy)essigsäure-(1,3-dimethyl-but-1-yl)-ester (S2-2), (5-Chlor-8-chinolinoxy)essigsäure-4-allyl-oxy-butylester (S2-3), (5-Chlor-8-chinolinoxy)essigsäure-1-allyloxy-prop-2-ylester (S2-4), (5-Chlor-8-chinolinoxy)essigsäureethylester (S2-5), (5-Chlor-8-chinolinoxy)essigsäuremethylester (S2-6), (5-Chlor-8-chinolinoxy)essigsäureallylester (S2-7), (5-Chlor-8-chinolinoxy)essigsäure-2-(2-propyliden-iminoxy)-1-ethylester (S2-8), (5-Chlor-8-chinolinoxy)essigsäure-2-oxo-prop-1-ylester (S2-9) und verwandte Verbindungen, wie sie in EP-A-86 750, EP-A-94 349 und EP-A-191 736 oder EP-A-0 492 366 beschrieben sind, sowie (5-Chlor-8-chinolinoxy)essigsäure (S2-10), deren Hydrate und Salze, beispielsweise deren Lithium-, Natrium- Kalium-, Kalzium-, Magnesium-, Aluminium-, Eisen-, Ammonium-, quartäre Ammonium-, Sulfonium-, oder Phosphoniumsalze wie sie in der WO-A-2002/34048 beschrieben sind;
   S2^{b}) Verbindungen vom Typ der (5-Chlor-8-chinolinoxy)malonsäure (S2^{b}), vorzugsweise Verbindungen wie (5-Chlor-8-chinolinoxy)malonsäurediethylester, (5-Chlor-8-chinolinoxy)malonsäurediallylester, (5-Chlor-8-chinolinoxy)malonsäure-methyl-ethylester und verwandte Verbindungen, wie sie in EP-A-0 582 198 beschrieben sind.
S3) Wirkstoffe vom Typ der Dichloracetamide (S3), die häufig als Vorauflaufsafener (bodenwirksame Safener) angewendet werden, wie z. B.
   "Dichlormid" (N,N-Diallyl-2,2-dichloracetamid) (S3-1),
   "R-29148" (3-Dichloracetyl-2,2,5-trimethyl-1,3-oxazolidin) der Firma Stauffer (S3-2),
   "R-28725" (3-Dichloracetyl-2,2,-dimethyl-1,3-oxazolidin) der Firma Stauffer (S3-3),
   "Benoxacor" (4-Dichloracetyl-3,4-dihydro-3-methyl-2H-1,4-benzoxazin) (S3-4),
   "PPG-1292" (N-Allyl-N-[(1,3-dioxolan-2-yl)-methyl]-dichloracetamid) der Firma PPG Industries (S3-5),
   "DKA-24" (N-Allyl-N-[(allylaminocarbonyl)methyl]-dichloracetamid) der Firma Sagro-Chem (S3-6),
   "AD-67" oder "MON 4660" (3-Dichloracetyl-1-oxa-3-aza-spiro[4,5]decan) der Firma Nitrokemia bzw. Monsanto (S3-7),
   "TI-35" (1-Dichloracetyl-azepan) der Firma TRI-Chemical RT (S3-8),
   "Diclonon" (Dicyclonon) oder "BAS145138" oder "LAB145138" (S3-9) (3-Dichloracetyl-2,5,5-trimethyl-1,3-diazabicyclo[4.3.0]nonan) der Firma BASF,
   "Furilazol" oder "MON 13900" ((RS)-3-Dichloracetyl-5-(2-furyl)-2,2-dimethyl-oxazolidin) (S3-10), sowie dessen (R)-Isomer (S3-11).
S4) Verbindungen aus der Klasse der Acylsulfonamide (S4):
   S4^{a}) N-Acylsulfonamide der Formel (S4^{a}) und deren Salze wie sie in der
      WO-A-97/45016 beschrieben sind, 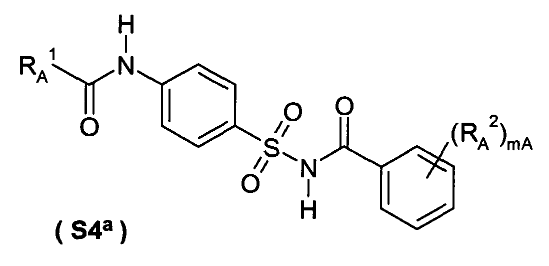 worin
      R_{A}¹ (C₁-C₆)Alkyl, welches durch v_{A} Substituenten aus der Gruppe Halogen, (C₁-C₄)Alkoxy, (C₁-C₆)Haloalkoxy und (C₁-C₄)Alkylthio und im Falle cyclischer Reste auch durch (C₁-C₄)Alkyl und (C₁-C₄)Haloalkyl substituiert ist;
      R_{A}² Halogen, (C₁-C₄)Alkyl, (C₁-C₄)Alkoxy, CF_{3;}
      m_{A} 1 oder 2;
      v_{A} ist 0, 1, 2 oder 3 bedeuten;
   S4^{b}) Verbindungen vom Typ der 4-(Benzoylsulfamoyl)benzamide der Formel (S4^{b})
      und deren Salze, wie sie in der WO-A-99/16744 beschrieben sind, 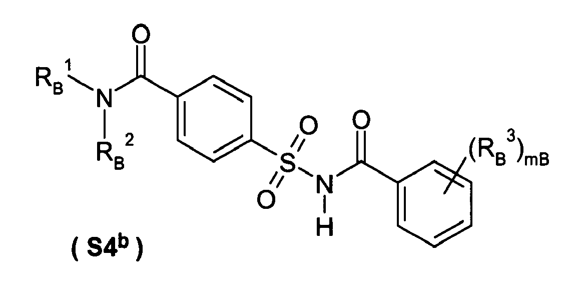 worin
      R_{B}¹, R_{B}² unabhängig voneinander Wasserstoff, (C₁-C₆)Alkyl, (C₃-C₆)Alkenyl, (C₃-C₆)Alkinyl,
      R_{B3} Halogen, (C₁-C₄)Alkyl, (C₁-C₄)Haloalkyl oder (C₁-C₄)Alkoxy und
      m_{B} 1 oder 2 bedeuten,
      z.B. solche worin
      R_{B}¹ = Cyclopropyl, R_{B}² = Wasserstoff und (R_{B}³) = 2-OMe ist (S4-1, "Cyprosulfamide",),
      R_{B}¹ = Cyclopropyl, R_{B}² = Wasserstoff und (R_{B}³) = 5-Cl-2-OMe ist (S4-2),
      R_{B}¹ = Ethyl, R_{B}² = Wasserstoff und (R_{B3}) = 2-OMe ist (S4-₃),
      R_{B}¹ = Isopropyl, R_{B}² = Wasserstoff und (R_{B}³) = 5-Cl-2-OMe ist (S4-4) und
      R_{B}¹ = Isopropyl, R_{B}² = Wasserstoff und (R_{B}³) = 2-OMe ist (S4-5).
   S4^{c}) Verbindungen aus der Klasse der Benzoylsulfamoylphenylharnstoffe der Formel (S4^{c}), wie sie in der EP-A-365484 beschrieben sind 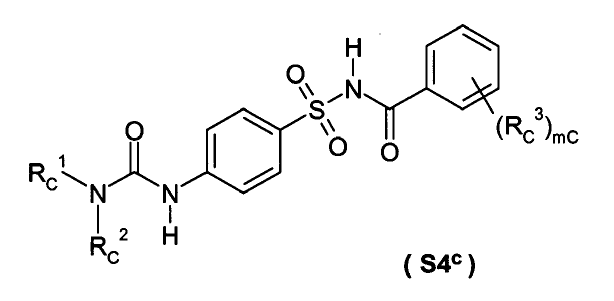 worin
      R_{C}¹, R_{C}² unabhängig voneinander Wasserstoff, (C₁-C₈)Alkyl, (C₃-C₆)Alkenyl, (C₃-C₆)Alkinyl,
      R_{C}³ Halogen, (C₁-C₄)Alkyl, (C₁-C₄)Alkoxy, CF₃
      m_{C} 1 oder 2 bedeuten;
      beispielsweise
      1-[4-(N-2-Methoxybenzoylsulfamoyl)phenyl]-3-methylharnstoff, 1-[4-(N-2-Methoxybenzoylsulfamoyl)phenyl]-3,3-dimethylharnstoff, 1-[4-(N-4,5-Dimethylbenzoylsulfamoyl)phenyl]-3-methylharnstoff.
S5) Wirkstoffe aus der Klasse der Hydroxyaromaten und der aromatischaliphatischen Carbonsäurederivate (S5), z.B.
   3,4,5-Triacetoxybenzoesäureethylester, 3,5-Dimethoxy-4-hydroxybenzoesäure, 3,5-Dihydroxybenzoesäure, 4-Hydroxysalicylsäure, 4-Fluorsalicyclsäure, 2-Hydroxyzimtsäure, 2,4-Dichlorzimtsäure, wie sie in der WO-A-2004/084631, WO-A-2005/015994, WO-A-2005/016001 beschrieben sind.
S6) Wirkstoffe aus der Klasse der 1,2-Dihydrochinoxalin-2-one (S6), z.B. 1-Methyl-3-(2-thienyl)-1,2-dihydrochinoxalin-2-on, 1-Methyl-3-(2-thienyl)-1,2-dihydrochinoxalin-2-thion, 1-(2-Aminoethyl)-3-(2-thienyl)-1,2-dihydrochinoxalin-2-on-hydrochlorid, 1-(2-Methylsulfonylaminoethyl)-3-(2-thienyl)-1,2-dihydro-chinoxalin-2-on, wie sie in der WO-A-2005/112630 beschrieben sind.
S7) Verbindungen aus der Klasse der Diphenylmethoxyessigsäurederivate (S7), z.B. Diphenylmethoxyessigsäuremethylester (CAS-Reg.Nr. 41858-19-9) (S7-1), Diphenylmethoxyessigsäureethylester oder Diphenylmethoxyessigsäure wie sie in der WO-A-98/38856 beschrieben sind.
S8) Verbindungen der Formel (S8),wie sie in der WO-A-98/27049 beschrieben sind 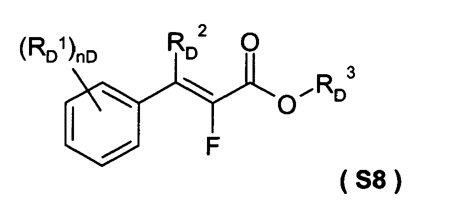 worin die Symbole und Indizes folgende Bedeutungen haben:
   R_{D}¹ ist Halogen, (C₁-C₄)Alkyl, (C₁-C₄)Haloalkyl, (C₁-C₄)Alkoxy, (C₁-C₄)Haloalkoxy,
   R_{D}² ist Wasserstoff oder (C₁-C₄)Alkyl
   R_{D3} ist Wasserstoff, (C₁-C₈)Alkyl, (C₂-C₄)Alkenyl, (C₂-C₄)Alkinyl, oder Aryl,
   wobei jeder der vorgenannten C-haltigen Reste unsubstituiert oder
   durch einen oder mehrere, vorzugsweise bis zu drei gleiche oder
   verschiedene Reste aus der Gruppe, bestehend aus Halogen und
   Alkoxy substituiert ist; oder deren Salze
   n_{D} ist eine ganze Zahl von 0 bis 2.
S9) Wirkstoffe aus der Klasse der 3-(5-Tetrazolylcarbonyl)-2-chinolone (S9), z.B. 1,2-Dihydro-4-hydroxy-1-ethyl-3-(5-tetrazolylcarbonyl)-2-chinolon (CAS-Reg. Nr. 219479-18-2), 1,2-Dihydro-4-hydroxy-1-methyl-3-(5-tetrazolylcarbonyl)-2-chinolon (CAS-Reg. Nr. 95855-00-8), wie sie in der WO-A-1999/000020 beschrieben sind.
S10) Verbindungen der Formeln (S10^{a}) oder (S10^{b})
   wie sie in der WO-A-2007/023719 und WO-A-2007/023764 beschrieben sind

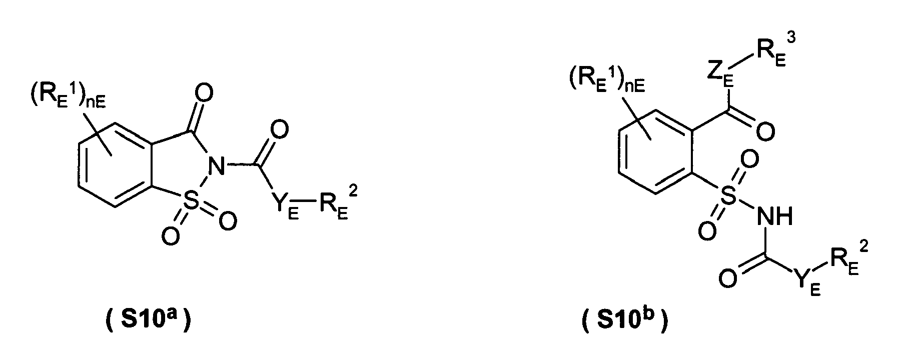

   worin
   R_{E}1 Halogen, (C₁-C₄)Alkyl, Methoxy, Nitro, Cyano, CF₃, OCF₃
   Y_{E}, Z_{E} unabhängig voneinander O oder S,

   n_{E} eine ganze Zahl von 0 bis 4,
   R_{E}² (C₁-C₁₆)Alkyl, (C₂-C₆)Alkenyl, Aryl, Benzyl, Halogenbenzyl,
   R_{E}³ Wasserstoff oder (C₁-C₆)Alkyl bedeuten.
S11) Wirkstoffe vom Typ der Oxyimino-Verbindungen (S11), die als Saatbeizmittel bekannt sind, wie z. B.
   "Oxabetrinil" ((Z)-1,3-Dioxolan-2-ylmethoxyimino (phenyl)acetonitril) (S11-1), das als Saatbeiz-Safener für Hirse gegen Schäden von Metolachlor bekannt ist,
   "Fluxofenim" (1-(4-Chlorphenyl)-2,2,2-trifluor-1-ethanon-O-(1,3-dioxolan-2-ylmethyl)-oxim) (S11-2), das als Saatbeiz-Safener für Hirse gegen Schäden von Metolachlor bekannt ist, und
   "Cyometrinil" oder "CGA-43089" ((Z)-Cyanomethoxyimino(phenyl)acetonitril) (S11-3), das als Saatbeiz-Safener für Hirse gegen Schäden von Metolachlor bekannt ist.
S12) Wirkstoffe aus der Klasse der Isothiochromanone (S12), wie z.B. Methyl-[(3-oxo-1 H-2-benzothiopyran-4(3H)-yliden)methoxy]acetate (CAS-Reg. Nr. 205121-04-6) (S12-1) und verwandte Verbindungen aus WO-A-1998/13361.
S13) Eine oder mehrere Verbindungen aus Gruppe (S13):
   "Naphthalic anhydrid" (1,8-Naphthalindicarbonsäureanhydrid) (S13-1), das als Saatbeiz-Safener für Mais gegen Schäden von Thiocarbamatherbiziden bekannt ist,

   "Fenclorim" (4,6-Dichlor-2-phenylpyrimidin) (S13-2), das als Safener für Pretilachlor in gesätem Reis bekannt ist,
   "Flurazole" (Benzyl-2-chlor-4-trifluormethyl-1,3-thiazol-5-carboxylat) (S13-3), das als Saatbeiz-Safener für Hirse gegen Schäden von Alachlor und
   Metolachlor bekannt ist,
   "CL 304415" (CAS-Reg. Nr. 31541-57-8)
   (4-Carboxy-3,4-dihydro-2H-1-benzopyran-4-essigsäure) (S13-4) der Firma American Cyanamid, das als Safener für Mais gegen Schäden von Imidazolinonen bekannt ist,
   "MG 191" (CAS-Reg. Nr. 96420-72-3) (2-Dichlormethyl-2-methyl-1,3-dioxolan) (S13-5) der Firma Nitrokemia, das als Safener für Mais bekannt ist,
   "MG-838" (CAS-Reg. Nr. 133993-74-5)
   (2-propenyl 1-oxa-4-azaspiro[4.5]decane-4-carbodithioate) (S13-6) der Firma Nitrokemia
   "Disulfoton" (O,O-Diethyl S-2-ethylthioethyl phosphordithioat) (S13-7), "Dietholate" (O,O-Diethyl-O-phenylphosphorotioat) (S13-8),
   "Mephenate" (4-Chlorphenyl-methylcarbamat) (S13-9).
S14) Wirkstoffe, die neben einer herbiziden Wirkung gegen Schadpflanzen auch Safenerwirkung an Kulturpflanzen wie Reis aufweisen, wie z. B.
   "Dimepiperate" oder "MY-93" (*S*-1-Methyl-1-phenylethyl-piperidin-1-carbothioat), das als Safener für Reis gegen Schäden des Herbizids Molinate bekannt ist,
   "Daimuron" oder "SK 23" (1-(1-Methyl-1-phenylethyl)-3-p-tolyl-harnstoff), das als Safener für Reis gegen Schäden des Herbizids Imazosulfuron bekannt ist,
   "Cumyluron" = "JC-940" (3-(2-Chlorphenylmethyl)-1-(1-methyl-1-phenylethyl)harnstoff, siehe JP-A-60087254), das als Safener für Reis gegen Schäden einiger Herbizide bekannt ist,
   "Methoxyphenon" oder "NK 049" (3,3'-Dimethyl-4-methoxy-benzophenon), das als Safener für Reis gegen Schäden einiger Herbizide bekannt ist,
   "CSB" (1-Brom-4-(chlormethylsulfonyl)benzol) von Kumiai, (CAS-Reg. Nr. 54091-06-4), das als Safener gegen Schäden einiger Herbizide in Reis bekannt ist.
S15) Wirkstoffe, die vorrangig als Herbizide eingesetzt werden, jedoch auch Safenerwirkung auf Kulturpflanzen aufweisen, z.B.
   (2,4-Dichlorphenoxy)essigsäure (2,4-D),
   (4-Chlorphenoxy)essigsäure,
   (R,S)-2-(4-Chlor-o-tolyloxy)propionsäure (Mecoprop),
   4-(2,4-Dichlorphenoxy)buttersäure (2,4-DB),
   (4-Chlor-o-tolyloxy)essigsäure (MCPA),
   4-(4-Chlor-o-tolyloxy)buttersäure,
   4-(4-Chlorphenoxy)buttersäure,
   3,6-Dichlor-2-methoxybenzoesäure (Dicamba),
   1-(Ethoxycarbonyl)ethyl-3,6-dichlor-2-methoxybenzoat (Lactidichlor-ethyl).

Einige der Safener sind auch als Herbizide bekannt und entfalten somit neben der Herbizidwirkung bei Schadpflanzen zugleich auch Schutzwirkung bei den Kulturpflanzen.

Die Gewichtsverhältnisse von Herbizid(mischung) zu Safener hängt im Allgemeinen von der Aufwandmenge an Herbizid und der Wirksamkeit des jeweiligen Safeners ab und kann innerhalb weiter Grenzen variieren, beispielsweise im Bereich von 200:1 bis 1:200, vorzugsweise 100:1 bis 1:100, insbesondere 20:1 bis 1:20. Die Safener können analog den erfindungsgemäßen Verbindungen oder deren Mischungen mit weiteren Pestiziden formuliert werden und als Fertigformulierung oder Tankmischung mit den erfindungsgemäßen Verbindungen bereitgestellt und angewendet werden.

Zur Anwendung werden die in handelsüblicher Form vorliegenden Formulierungen gegebenenfalls in üblicher Weise verdünnt z. B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der erfindungsgemäßen Verbindungen. Sie kann innerhalb weiter Grenzen schwanken, z. B. zwischen 0,001 und 10,0 kg/ha oder mehr Aktivsubstanz, vorzugsweise liegt sie jedoch zwischen 0,005 und 5 kg/ha.

### Beispiele

### A. Synthesebeispiele

Nachfolgend sind Synthesen erfindungsgemäßer Verbindungen beispielhaft beschrieben, ohne dass diese Beispiele limitierenden Charakter haben.
1. (6-Amino-5-chlor-2-(2,6-dichlorphenyl)pyrimidin-4-yl)propandinitril (Bsp. Nr. 174) Stufe A:
   Zu einer Lösung von 72 mg Malondinitril in 10 ml THF wird bei 0°C Natriumhydrid (45 mg als 60%ige Suspension in Mineralöl) zugegeben und die Mischung anschließend für 30 Minuten bei Raumtemperatur gerührt.
   4,5,6-Trichlor-2-(2,6-dichlorphenyl)pyrimidin (0,30 g; analog folgender Vorschrift hergestellt: K. Findeisen, K. Wagner, Synthesis 1978, 40-42) wird zugegeben und die Reaktionsmischung bei Raumtemperatur über Nacht gerührt. Der Ansatz wird auf 10 ml gesättigte Ammoniumchlorid-Lösung gegeben, mehrmals mit Ethylacetat extrahiert und die vereinigten organischen Phasen mit gesättigter Natriumchlorid-Lösung gewaschen. Nach dem Trocknen über Magnesiumsulfat wird das Lösungsmittel im Vakuum eingeengt, wodurch Verbindung Nr. 505 erhalten wird.
   Stufe B:
   Das erhaltene Produkt wird in 5 ml konzentrierter Ammoniaklösung suspendiert und 8 Stunden lang bei 150°C im geschlossenen Gefäß in einem Mikrowellengerät zur Reaktion gebracht. Die Lösung wird anschließend im Vakuum auf das halbe Volumen eingeengt und mit 2N Salzsäure angesäuert.
   Der ausgefallene Feststoff wird mit Wasser und Ethylacetat gewaschen und
      anschließend getrocknet. Es werden 0,19 g (61% der Theorie) des gewünschten Produkts erhalten.
2. [6-Amino-5-chlor-2-phenylpyrimidin-4-yl]propandinitril (Bsp. Nr. 1)
   Die Substanz wird analog der Vorschrift zu Bsp. Nr. 174 dargestellt. Die Ausgangssubstanz, 4,5,6-Trichlor-2-phenylpyrimidin erhält man nach literaturbekannter Methode: K. Findeisen, K. Wagner, Synthesis 1978, 40-42.
3. [6-Amino-5-chlor-2-(4-chlorphenyl)pyrimidin-4-yl]propandinitril (Bsp. Nr. 29)
   Die Substanz wird analog der Vorschrift zu Bsp. Nr. 174 dargestellt. Die Ausgangssubstanz, 4,5,6-Trichlor-2-(4-chlorphenyl)pyrimidin erhält man nach literaturbekannter Methode: K. Findeisen, K. Wagner, Synthesis 1978, 40-42.
4. [5-Chlor-2-(4-chlorphenyl)-6-(methylamino)pyrimidin-4-yl]propandinitril (Bsp. Nr. 41)
   Die Substanz erhält man analog der Vorschrift zu Bsp. Nr. 174. In der Stufe B wird jedoch Methylamin (40% Lösung in MeOH) eingesetzt und die Reaktion bei 120°C für eine Stunde im Mikrowellengerät durchgeführt.
5. [6-Amino-5-chlor-2-(3-fluorphenyl)pyrimidin-4-yl]propandinitril (Bsp. Nr. 80)
   Die Substanz wird analog der Vorschrift zu Bsp. Nr. 174 dargestellt. Die Ausgangssubstanz, 4,5,6-Trichlor-2-(3-fluorphenyl)pyrimidin erhält man analog der literaturbekannten Methode: K. Findeisen, K. Wagner, Synthesis 1978, 40-42.
6. (6-Amino-5-chlor-2-methylpyrimidin-4-yl)propandinitril (Bsp. Nr. 261)
   Die Substanz wird analog der Vorschrift zu Bsp. Nr. 174 dargestellt. Die Ausgangssubstanz, 4,5,6-Trichlor-2-methylpyrimidin erhält man nach literaturbekannter Methode: H. Gershon, K. Dittmer, R. Braun, J. Org. Chem. 1961, 26, 1874-1877.
7. (6-Amino-5-chlor-2-tert-butylpyrimidin-4-yl)propandinitril (Bsp. Nr. 266)
   Die Substanz wird analog der Vorschrift zu Bsp. Nr. 174 dargestellt. Die Ausgangssubstanz, 4,5,6-Trichlor-2-tert-butylpyrimidin erhält man in Analogie zur literaturbekannten Methode: H. Gershon, K. Dittmer, R. Braun, J. Org. Chem. 1961,26,1874-1877.
8. Ammonium-[6-amino-5-chlor-2-(2,6-dichlorphenyl)pyrimidin-4-yl](dicyan)methanid (Bsp. Nr. 360)
   Es werden 100 mg der Verbindung Bsp. Nr. 174 in Ammoniaklösung (2M in EtOH, 2ml) vollständig gelöst, im Vakuum bei 40°C eingeengt und der erhaltene Feststoff getrocknet. Es werden 104 mg des gewünschten Produkts erhalten.
9. Ammonium-[6-amino-5-chlor-2-phenylpyrimidin-4-yl](dicyan)methanid (Bsp. Nr. 277)
   Die Substanz wird analog der Vorschrift zu Bsp. Nr. 360 dargestellt.
10. Natrium-[6-amino-5-chlor-2-(4-chlorphenyl)-pyrimidin-4-yl](dicyan)methanid (Bsp. Nr. 293)
   Durch Zugabe einer stöchiometrischen Menge von Natriummethylat zur in Ethanol gelösten Verbindung Bsp. Nr. 29 kann das entsprechende Natriumsalz nach dem Einengen im Vakuum und anschließendem Trocknen erhalten werden.
11. Ammonium-[6-amino-5-chlor-2-(4-chlorphenyl)-pyrimidin-4-yl](dicyan)methanid (Bsp. Nr. 297)
   Die Substanz wird analog der Vorschrift zu Bsp. Nr. 360 dargestellt.
12. Ammonium-[6-amino-5-chlor-2-(3-fluorphenyl)-pyrimidin-4-yl](dicyan)methanid (Bsp. Nr. 321)
   Die Substanz wird analog der Vorschrift zu Bsp. Nr. 360 dargestellt.

Die in den nachfolgenden Tabellen 1-4 beschriebenen Verbindungen erhält man analog zu den oben beschriebenen Synthesebeispielen. Die in Tabelle 5 beschriebenen Verbindungen erhält man analog zu Synthesebeispiel A1, Stufe A.

In den Tabellen 1-5 bedeuten:
Me = Methyl
Et = Ethyl
cBu = Cyclobutyl
cPr = Cyclopropyl
iPr = Isopropyl
cHex = Cyclohexyl
tBu = tert-Butyl
Ph = Phenyl
Vin = Vinyl
Ac = Acetyl
Hal = Halogen

**Tabelle 1: Verbindungen der Formel (I)**

| | | | | |
|---|---|---|---|---|
| 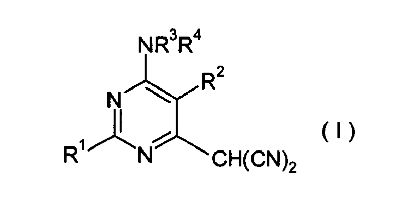 | | | | |

| Bsp. Nr. | R¹ | R² | R³ | R⁴ |
|---|---|---|---|---|
| 1. | Ph | Cl | H | H |
| 2. | Ph | Br | H | H |
| 3. | Ph | Cl | H | Ac |
| 4. | Ph | Br | H | Ac |
| 5. | Ph | Cl | H | Me |
| 6. | Ph | Br | H | Me |
| 7. | 4-OMe-Ph | Cl | H | H |
| 8. | 4-OMe-Ph | Br | H | H |
| 9. | 4-NO₂-Ph | Cl | H | H |
| 10. | 4-NO₂-Ph | Br | H | H |
| 11. | 4-Me-Ph | F | H | H |
| 12. | 4-Me-Ph | Cl | H | H |
| 13. | 4-Me-Ph | Br | H | H |
| 14. | 4-I-Ph | Cl | H | H |
| 15. | 4-I-Ph | Br | H | H |
| 16. | 4-I-Ph | Cl | H | Ac |
| 17. | 4-I-Ph | Br | H | Ac |
| 18. | 4-I-Ph | Cl | H | Me |
| 19. | 4-I-Ph | Br | H | Me |
| 20. | 4-F-Ph | Cl | H | H |
| 21. | 4-F-Ph | Br | H | H |
| 22. | 4-F-Ph | Cl | H | Ac |
| 23. | 4-F-Ph | Br | H | Ac |
| 24. | 4-F-Ph | Cl | H | Me |
| 25. | 4-F-Ph | Br | H | Me |
| 26. | 4-COOMe-Ph | Cl | H | H |
| 27. | 4-COOMe-Ph | Br | H | H |
| 28. | 4-Cl-Ph | F | H | H |
| 29. | 4-Cl-Ph | Cl | H | H |
| 30. | 4-Cl-Ph | Br | H | H |
| 31. | 4-Cl-Ph | I | H | H |
| 32. | 4-Cl-Ph | CN | H | H |
| 33. | 4-Cl-Ph | NO₂ | H | H |
| 34. | 4-Cl-Ph | NO₂ | H | Me |
| 35. | 4-Cl-Ph | Cl | =CHN(Me)₂ | |
| 36. | 4-Cl-Ph | Br | =CHN(Me)₂ | |
| 37. | 4-Cl-Ph | Cl | H | Ac |
| 38. | 4-Cl-Ph | Br | H | Ac |
| 39. | 4-Cl-Ph | Cl | H | Et |
| 40. | 4-Cl-Ph | Br | H | Et |
| 41. | 4-Cl-Ph | Cl | H | Me |
| 42. | 4-Cl-Ph | Br | H | Me |
| 43. | 4-Cl-Ph | Cl | Me | Me |
| 44. | 4-Cl-Ph | Br | Me | Me |
| 45. | 4-CF3-Ph | Cl | H | H |
| 46. | 4-CF3-Ph | Br | H | H |
| 47. | 4-CF3-Ph | Cl | H | Ac |
| 48. | 4-CF3-Ph | Br | H | Ac |
| 49. | 4-CF3-Ph | Cl | H | Me |
| 50. | 4-CF3-Ph | Br | H | Me |
| 51. | 4-Br-Ph | F | H | H |
| 52. | 4-Br-Ph | Cl | H | H |
| 53. | 4-Br-Ph | Br | H | H |
| 54. | 4-Br-Ph | I | H | H |
| 55. | 4-Br-Ph | CN | H | H |
| 56. | 4-Br-Ph | NO₂ | H | H |
| 57. | 4-Br-Ph | Cl | =CHN(Me)₂ | |
| 58. | 4-Br-Ph | Br | =CHN(Me)₂ | |
| 59. | 4-Br-Ph | Cl | H | Ac |
| 60. | 4-Br-Ph | Br | H | Ac |
| 61. | 4-Br-Ph | Cl | H | Et |
| 62. | 4-Br-Ph | Br | H | Et |
| 63. | 4-Br-Ph | Cl | H | Me |
| 64. | 4-Br-Ph | Br | H | Me |
| 65. | 4-Br-Ph | Cl | Me | Me |
| 66. | 4-Br-Ph | Br | Me | Me |
| 67. | 3-OMe-4-F-Ph | F | H | H |
| 68. | 3-OMe-4-F-Ph | Cl | H | H |
| 69. | 3-OMe-4-F-Ph | Br | H | H |
| 70. | 3-OMe-4-Cl-Ph | F | H | H |
| 71. | 3-OMe-4-Cl-Ph | Cl | H | H |
| 72. | 3-OMe-4-Cl-Ph | Br | H | H |
| 73. | 3-Me-Ph | Cl | H | H |
| 74. | 3-Me-Ph | Br | H | H |
| 75. | 3-Me-Ph | F | H | H |
| 76. | 3-Me-4-C-Ph | F | H | H |
| 77. | 3-Me-4-Cl-Ph | Cl | H | H |
| 78. | 3-Me-4-Cl-Ph | Br | H | H |
| 79. | 3-F-Ph | F | H | H |
| 80. | 3-F-Ph | Cl | H | H |
| 81. | 3-F-Ph | Br | H | H |
| 82. | 3-Cl-Ph | Cl | H | H |
| 83. | 3-Cl-Ph | Br | H | H |
| 84. | 3-CF₃-Ph | F | H | H |
| 85. | 3-CF₃-Ph | Cl | H | H |
| 86. | 3-CF₃-Ph | Br | H | H |
| 87. | 3-CF₃-4-Cl-Ph | Cl | H | H |
| 88. | 3-CF₃-4-Cl-Ph | Br | H | H |
| 89. | 3-CF₃-4-Cl-Ph | F | H | H |
| 90. | 3-CF₃-4-Cl-Ph | I | H | H |
| 91. | 3,6-di-Cl-Ph | F | H | H |
| 92. | 3,6-di-Cl-Ph | Cl | H | H |
| 93. | 3,6-di-Cl-Ph | Br | H | H |
| 94. | 3,5-di-Cl-Ph | F | H | H |
| 95. | 3,5-di-Cl-Ph | Cl | H | H |
| 96. | 3,5-di-Cl-Ph | Br | H | H |
| 97. | 3,5-di-CF₃-Ph | F | H | H |
| 98. | 3,5-di-CF₃-Ph | Cl | H | H |
| 99. | 3,5-di-CF₃-Ph | Br | H | H |
| 100. | 3,4-di-OMe-Ph | F | H | H |
| 101. | 3,4-di-OMe-Ph | Cl | H | H |
| 102. | 3,4-di-OMe-Ph | Br | H | H |
| 103. | 3,4-di-Cl-Ph | F | H | H |
| 104. | 3,4-di-Cl-Ph | Cl | H | H |
| 105. | 3,4-di-Cl-Ph | Br | H | H |
| 106. | 2-F-6-Cl-Ph | F | H | H |
| 107. | 2-F-6-Cl-Ph | Cl | H | H |
| 108. | 2-F-6-Cl-Ph | Br | H | H |
| 109. | 2-F-4-Cl-Ph | F | H | H |
| 110. | 2-F-4-Cl-Ph | Cl | H | H |
| 111. | 2-F-4-Cl-Ph | Br | H | H |
| 112. | 2-F-4-Cl-5-OMe-Ph | Cl | H | H |
| 113. | 2-F-4-Cl-5-OMe-Ph | Br | H | H |
| 114. | 2-F-4-Cl-5-OMe-Ph | Cl | H | Ac |
| 115. | 2-F-4-Cl-5-OMe-Ph | Br | H | Ac |
| 116. | 2-F-4-Cl-5-OMe-Ph | Cl | H | Me |
| 117. | 2-F-4-Cl-5-OMe-Ph | Br | H | Me |
| 118. | 2-F-4,5-di-Cl-Ph | F | H | H |
| 119. | 2-F-4,5-di-Cl-Ph | Cl | H | H |
| 120. | 2-F-4,5-di-Cl-Ph | Br | H | H |
| 121. | 2-F-3-SMe-4-Cl-Ph | Cl | H | H |
| 122. | 2-F-3-SMe-4-Cl-Ph | Br | H | H |
| 123. | 2-F-3-SMe-4-Cl-Ph | Cl | H | Ac |
| 124. | 2-F-3-SMe-4-Cl-Ph | Br | H | Ac |
| 125. | 2-F-3-SMe-4-Cl-Ph | Cl | H | Me |
| 126. | 2-F-3-SMe-4-Cl-Ph | Br | H | Me |
| 127. | 2-F-3-SCF3-4-Cl-Ph | F | H | H |
| 128. | 2-F-3-SCF3-4-Cl-Ph | Cl | H | H |
| 129. | 2-F-3-SCF3-4-Cl-Ph | Br | H | H |
| 130. | 2-F-3-S(O₂)Me-4-Cl-Ph | F | H | H |
| 131. | 2-F-3-S(O₂)Me-4-Cl-Ph | Cl | H | H |
| 132. | 2-F-3-S(O₂)Me-4-Cl-Ph | Br | H | H |
| 133. | 2-F-3-S(O)Me-4-Cl-Ph | F | H | H |
| 134. | 2-F-3-S(O)Me-4-Cl-Ph | Cl | H | H |
| 135. | 2-F-3-S(O)Me-4-Cl-Ph | Br | H | H |
| 136. | 2-F-3-OMe-4-Cl-Ph | F | H | H |
| 137. | 2-F-3-OMe-4-Cl-Ph | Cl | H | H |
| 138. | 2-F-3-OMe-4-Cl-Ph | Br | H | H |
| 139. | 2-F-3-OMe-4-Cl-Ph | I | H | H |
| 140. | 2-F-3-OMe-4-Cl-Ph | CN | H | H |
| 141. | 2-F-3-OMe-4-Cl-Ph | NO₂ | H | H |
| 142. | 2-F-3-OMe-4-Cl-Ph | Cl | =CHN(Me)₂ | |
| 143. | 2-F-3-OMe-4-Cl-Ph | Br | =CHN(Me)₂ | |
| 144. | 2-F-3-OMe-4-Cl-Ph | Cl | H | Ac |
| 145. | 2-F-3-OMe-4-Cl-Ph | Br | H | Ac |
| 146. | 2-F-3-OMe-4-Cl-Ph | Cl | H | Et |
| 147. | 2-F-3-OMe-4-Cl-Ph | Br | H | Et |
| 148. | 2-F-3-OMe-4-Cl-Ph | Cl | H | Me |
| 149. | 2-F-3-OMe-4-Cl-Ph | Br | H | Me |
| 150. | 2-F-3-OMe-4-Cl-Ph | Cl | Me | Me |
| 151. | 2-F-3-OMe-4-Cl-Ph | Br | Me | Me |
| 152. | 2-F-3-OEt-4-Cl-Ph | Cl | H | H |
| 153. | 2-F-3-OEt-4-Cl-Ph | Br | H | H |
| 154. | 2-F-3-OEt-4-Cl-Ph | Cl | H | Ac |
| 155. | 2-F-3-OMt-4-Cl-Ph | Br | H | Ac |
| 156. | 2-F-3-OEt-4-Cl-Ph | Cl | H | Me |
| 157. | 2-F-3-OEt-4-Cl-Ph | Br | H | Me |
| 158. | 2-F-3-OCF3-4-Cl-Ph | F | H | H |
| 159. | 2-F-3-OCF3-4-Cl-Ph | Cl | H | H |
| 160. | 2-F-3-OCF3-4-Cl-Ph | Br | H | H |
| 161. | 2-F-3-NMe2-4-Cl-Ph | F | H | H |
| 162. | 2-F-3-NMe2-4-Cl-Ph | Cl | H | H |
| 163. | 2-F-3-NMe2-4-Cl-Ph | Br | H | H |
| 164. | 2-F-3-Me-4-Cl-Ph | F | H | H |
| 165. | 2-F-3-Me-4-Cl-Ph | Cl | H | H |
| 166. | 2-F-3-Me-4-Cl-Ph | Br | H | H |
| 167. | 2-Cl-Ph | F | H | H |
| 168. | 2-Cl-Ph | Cl | H | H |
| 169. | 2-Cl-Ph | Br | H | H |
| 170. | 2,6-di-F-3-OMe-4-Cl-Ph | F | H | H |
| 171. | 2,6-di-F-3-OMe-4-Cl-Ph | Cl | H | H |
| 172. | 2,6-di-F-3-OMe-4-Cl-Ph | Br | H | H |
| 173. | 2,6-di-Cl-Ph | F | H | H |
| 174. | 2,6-di-Cl-Ph | Cl | H | H |
| 175. | 2,g-di-Cl-Ph | Br | H | H |
| 176. | 2,5-di-Cl-Ph | F | H | H |
| 177. | 2,5-di-Cl-Ph | Cl | H | H |
| 178. | 2,5-di-Cl-Ph | Br | H | H |
| 179. | 2,4-di-F-5-OMe-Ph | F | H | H |
| 180. | 2,4-di-F-5-OMe-Ph | Cl | H | H |
| 181. | 2,4-di-F-5-OMe-Ph | Br | H | H |
| 182. | 2,4-di-F-3-OMe-Ph | F | H | H |
| 183. | 2,4_di-F-3-OMe-Ph | Cl | H | H |
| 184. | 2,4-di-F-3-OMe-Ph | Br | H | H |
| 185. | 2,4-di-Cl-Ph | F | H | H |
| 186. | 2,4-di-Cl-Ph | Cl | H | H |
| 187. | 2,4-di-Cl-Ph | Br | H | H |
| 188. | 2,4-di-Cl-Ph | F | H | Ac |
| 189. | 2,4-di-Cl-Ph | Cl | H | Ac |
| 190. | 2,4-di-Cl-Ph | Br | H | Ac |
| 191. | 2,4-di-Cl-5-F-Ph | F | H | H |
| 192. | 2,4-di-Cl-5-F-Ph | Cl | H | H |
| 193. | 2,4-di-Cl-5-F-Ph | Br | H | H |
| 194. | 2,4-di-Cl-3-OMe-Ph | F | H | H |
| 195. | 2,4-di-Cl-3-OMe-Ph | Cl | H | H |
| 196. | 2,4-di-Cl-3-OMe-Ph | Br | H | H |
| 197. | 2,4,6-tri-Cl-Ph | F | H | H |
| 198. | 2,4,6-tri-Cl-Ph | Cl | H | H |
| 199. | 2,4,6-tri-Cl-Ph | Br | H | H |

**Tabelle 2: Verbindungen der Formel (I)**

| | | | | |
|---|---|---|---|---|
| 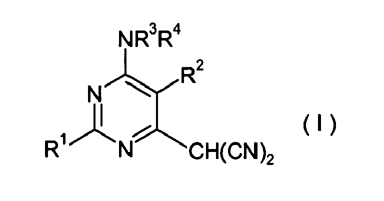 | | | | |

| Bsp. Nr. | R¹ | R² | R³ | R⁴ |
|---|---|---|---|---|
| 200. | 1-Cl-cPr | F | H | H |
| 201. | 1-Cl-cPr | Cl | H | H |
| 202. | 1-Cl-cPr | Br | H | H |
| 203. | 1-Cl-cPr | Cl | H | Ac |
| 204. | 1-Cl-cPr | Br | H | Ac |
| 205. | 1-Cl-cPr | Cl | H | Me |
| 206. | 1-Cl-cPr | Br | H | Me |
| 207. | 2-cPr-Vin | Cl | H | H |
| 208. | 2-cPr-Vin | Br | H | H |
| 209. | 2-di-Me-cPr | F | H | H |
| 210. | 2-di-Me-cPr | Cl | H | H |
| 211. | 2-di-Me-cPr | Br | H | H |
| 212. | 2-di-Me-cPr | Cl | H | Ac |
| 213. | 2-di-Me-cPr | Br | H | Ac |
| 214. | 2-di-Me-cPr | Cl | H | Me |
| 215. | 2-di-Me-cPr | Br | H | Me |
| 216. | 2-Me-Vin | Cl | H | H |
| 217. | 2-Me-Vin | Br | H | H |
| 218. | 2-Ph-Vin | Cl | H | H |
| 219. | 2-Ph-Vin | Br | H | H |
| 220. | 4-Cl-PhCH2 | Cl | H | H |
| 221. | 4-Cl-PhCHz | Br | H | H |
| 222. | cBu | F | H | H |
| 223. | cBu | Cl | H | H |
| 224. | cBu | Br | H | H |
| 225. | CF3 | F | H | H |
| 226. | CF3 | Cl | H | H |
| 227. | CF3 | Br | H | H |
| 228. | cHex | F | H | H |
| 229. | cHex | Cl | H | H |
| 230. | cHex | Br | H | H |
| 231. | cHex | Cl | H | Ac |
| 232. | cHex | Br | H | Ac |
| 233. | cHex | Cl | H | Me |
| 234. | cHex | Br | H | Me |
| 235. | cPr | Cl | =CHN(Me)₂ | |
| 236. | cpr | Br | =CHN(Me)₂ | |
| 237. | cPr | F | H | H |
| 238. | cPr | Cl | H | H |
| 239. | cPr | Br | H | H |
| 240. | cPr | I | H | H |
| 241. | cPr | CN | H | H |
| 242. | cPr | NO₂ | H | H |
| 243. | cPr | Cl | H | Ac |
| 244. | cPr | Br | H | Ac |
| 245. | cPr | Cl | H | Et |
| 246. | cPr | Br | H | Et |
| 247. | cPr | Cl | H | Me |
| 248. | cPr | Br | H | Me |
| 249. | cPr | Cl | Me | Me |
| 250. | cPr | Br | Me | Me |
| 251. | Hex | Cl | H | H |
| 252. | Hex | Br | H | H |
| 253. | iPr | F | H | H |
| 254. | iPr | Cl | H | H |
| 255. | iPr | Br | H | H |
| 256. | iPr | I | H | H |
| 257. | iPr | Cl | H | Ac |
| 258. | iPr | Br | H | Ac |
| 259. | iPr | Cl | H | Me |
| 260. | iPr | Br | H | Me |
| 261. | Me | Cl | H | H |
| 262. | Me | Br | H | H |
| 263. | PhCH₂ | Cl | H | H |
| 264. | PhCH₂ | Br | H | H |
| 265. | tBu | F | H | H |
| 266. | tBu | Cl | H | H |
| 267. | tBu | Br | H | H |
| 268. | tBu | I | H | H |
| 269. | Vin | F | H | H |
| 270. | Vin | Cl | H | H |
| 271. | Vin | Br | H | H |

**Tabelle 3: Salze der Verbindungen der Formel (I)**

| | | | | | |
|---|---|---|---|---|---|
| 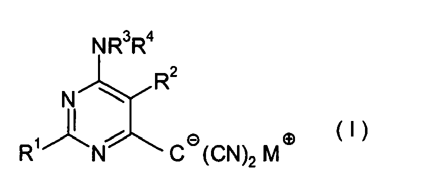 | | | | | |

| Bsp. Nr. | R¹ | R² | R³ | R⁴ | M^{⊕} |
|---|---|---|---|---|---|
| 272. | Ph | Cl | H | H | Li⁺ |
| 273. | Ph | Cl | H | H | Na⁺ |
| 274. | Ph | Cl | H | H | K⁺ |
| 275. | Ph | Cl | H | H | Ca²⁺ |
| 276. | Ph | Cl | H | H | Mg²⁺ |
| 277. | Ph | Cl | H | H | NH₄⁺ |
| 278. | Ph | Cl | H | H | NH₃Me⁺ |
| 279. | Ph | Cl | H | H | NH₂Me₂⁺ |
| 280. | Ph | Cl | H | H | NHMe₃⁺ |
| 281. | Ph | Cl | H | H | NMe₄⁺ |
| 282. | Ph | Br | H | H | NH₄⁺ |
| 283. | 4-OMe-Ph | Cl | H | H | NH₄⁺ |
| 284. | 4-NO₂-Ph | Cl | H | H | NH₄⁺ |
| 285. | 4-Me-Ph | Cl | H | H | NH₄⁺ |
| 286. | 4-I-Ph | Cl | H | H | NH₄⁺ |
| 287. | 4-F-Ph | Cl | H | H | Na⁺ |
| 288. | 4-F-Ph | Cl | H | H | K⁺ |
| 289. | 4-F-Ph | Cl | H | H | NH₄⁺ |
| 290. | 4-F-Ph | Cl | H | Ac | NH₄⁺ |
| 291. | 4-F-Ph | Cl | H | Me | NH₄⁺ |
| 292. | 4-Cl-Ph | Cl | H | H | Li⁺ |
| 293. | 4-Cl-Ph | Cl | H | H | Na⁺ |
| 294. | 4-Cl-Ph | Cl | H | H | K⁺ |
| 295. | 4-Cl-Ph | Cl | H | H | Ca²⁺ |
| 296. | 4-Cl-Ph | Cl | H | H | Mg²⁺ |
| 297. | 4-Cl-Ph | Cl | H | H | NH₄⁺ |
| 298. | 4-Cl-Ph | Cl | H | H | NH₃Me⁺ |
| 299. | 4-Cl-Ph | Cl | H | H | NH₂Me₂⁺ |
| 300. | 4-Cl-Ph | Cl | H | H | NHMe₃⁺ |
| 301. | 4-Cl-Ph | Cl | H | H | NMe₄⁺ |
| 302. | 4-Cl-Ph | Br | H | H | NH₄⁺ |
| 303. | 4-Cl-Ph | Cl | H | Ac | NH₄⁺ |
| 304. | 4-Cl-Ph | Cl | H | Et | NH₄⁺ |
| 305. | 4-Cl-Ph | Cl | H | Me | NH₄⁺ |
| 306. | 4-Cl-Ph | Cl | Me | Me | NH₄⁺ |
| 307. | 4-CF₃-Ph | Cl | H | H | NH₄⁺ |
| 308. | 4-Br-Ph | Cl | H | H | Na⁺ |
| 309. | 4-Br-Ph | Cl | H | H | K⁺ |
| 310. | 4-Br-Ph | Cl | H | H | NH₄⁺ |
| 311. | 4-Br-Ph | Br | H | H | Na⁺ |
| 312. | 4-Br-Ph | Br | H | H | K⁺ |
| 313. | 4-Br-Ph | Br | H | H | NH₄⁺ |
| 314. | 4-Br-Ph | Cl | H | Ac | NH₄⁺ |
| 315. | 4-Br-Ph | Cl | H | Et | NH₄⁺ |
| 316. | 4-Br-Ph | Cl | H | Me | NH₄⁺ |
| 317. | 3-OMe-4-F-Ph | Cl | H | H | Na⁺ |
| 318. | 3-OMe-4-F-Ph | Cl | H | H | NH₄⁺ |
| 319. | 3-Me-Ph | Cl | H | H | NH₄⁺ |
| 320. | 3-Me-4-Cl-Ph | Cl | H | H | NH₄⁺ |
| 321. | 3-F-Ph | Cl | H | H | NH₄⁺ |
| 322. | 3-Cl-Ph | Cl | H | H | NH₄⁺ |
| 323. | 3-CF₃-Ph | Cl | H | H | NH₄⁺ |
| 324. | 3-CF₃-4-Cl-Ph | Cl | H | H | NH₄⁺ |
| 325. | 3,6-di-Cl-Ph | Cl | H | H | NH₄⁺ |
| 326. | 3,5-di-Cl-Ph | Cl | H | H | NH₄⁺ |
| 327. | 3,5-di-CF₃-Ph | Cl | H | H | NH₄⁺ |
| 328. | 3,4-di-OMe-Ph | Cl | H | H | NH₄⁺ |
| 329. | 3,4-di-Cl-Ph | Cl | H | H | Na⁺ |
| 330. | 3,4-di-Cl-Ph | Cl | H | H | NH₄⁺ |
| 331. | 2-F-6-Cl-Ph | Cl | H | H | NH₄⁺ |
| 332. | 2-F-4-Cl-Ph | Cl | H | H | Na⁺ |
| 333. | 2-F-4-Cl-Ph | Cl | H | H | K⁺ |
| 334. | 2-F-4-Cl-Ph | Cl | H | H | NH₄⁺ |
| 335. | 2-F-4-Cl-5-OMe-Ph | Cl | H | H | Na⁺ |
| 336. | 2-F-4-Cl-5-OMe-Ph | Cl | H | H | NH₄⁺ |
| 337. | 2-F-4-Cl-5-OMe-Ph | Cl | H | Ac | NH₄⁺ |
| 338. | 2-F-4,5-di-Cl-Ph | Cl | H | H | NH₄⁺ |
| 339. | 2-F-3-SMe-4-Cl-Ph | Cl | H | H | NH₄⁺ |
| 340. | 2-F-3-SCF₃-4-Cl-Ph | Cl | H | H | NH₄⁺ |
| 341. | 2-F-3-S(O₂)Me-4-Cl-Ph | Cl | H | H | NH₄⁺ |
| 342. | 2-F-3-S(O)Me-4-Cl-Ph | Cl | H | H | NH₄⁺ |
| 343. | 2-F-3-OMe-4-Cl-Ph | Cl | H | H | Li⁺ |
| 344. | 2-F-3-OMe-4-Cl-Ph | Cl | H | H | Na⁺ |
| 345. | 2-F-3-OMe-4-Cl-Ph | Cl | H | H | K⁺ |
| 346. | 2-F-3-OMe-4-Cl-Ph | Cl | H | H | Ca²⁺ |
| 347. | 2-F-3-OMe-4-Cl-Ph | Cl | H | H | Mg²⁺ |
| 348. | 2-F-3-OMe-4-Cl-Ph | Cl | H | H | NH₄⁺ |
| 349. | 2-F-3-OMe-4-Cl-Ph | Cl | H | H | NH₃Me⁺ |
| 350. | 2-F-3-OMe-4-Cl-Ph | Cl | H | H | NH₂Me₂⁺ |
| 351. | 2-F-3-OMe-4-Cl-Ph | Cl | H | H | NHMe₃⁺ |
| 352. | 2-F-3-OMe-4-Cl-Ph | Cl | H | H | NMe₄⁺ |
| 353. | 2-F-3-OMe-4-Cl-Ph | Cl | H | Ac | NH₄⁺ |
| 354. | 2-F-3-OMe-4-Cl-Ph | Cl | H | Me | NH₄⁺ |
| 355. | 2-F-3-OEt-4-Cl-Ph | Cl | H | H | NH₄⁺ |
| 356. | 2-F-3-OCF₃-4-Cl-Ph | Cl | H | H | NH₄⁺ |
| 357. | 2-F-3-NMe₂-4-Cl-Ph | Cl | H | H | NH₄⁺ |
| 358. | 2-Cl-Ph | Cl | H | H | NH₄⁺ |
| 359. | 2,6-di-F-3-OMe-4-Cl-Ph | Cl | H | H | NH₄⁺ |
| 360. | 2,6-di-Cl-Ph | Cl | H | H | NH₄⁺ |
| 361. | 2,5-di-Cl-Ph | Cl | H | H | NH₄⁺ |
| 362. | 2,4-di-F-5-OMe-Ph | Cl | H | H | NH₄⁺ |
| 363. | 2,4-di-F-3-OMe-Ph | Cl | H | H | NH₄⁺ |
| 364. | 2,4-di-Cl-Ph | Cl | H | H | NH₄⁺ |
| 365. | 2,4-di-Cl-Ph | Cl | H | H | NH₄⁺ |
| 366. | 2,4-di-Cl-5-F-Ph | Cl | H | H | NH₄⁺ |
| 367. | 2,4-di-Cl-3-OMe-Ph | Cl | H | H | NH₄⁺ |
| 368. | 2,4,6-tri-Cl-Ph | Cl | H | H | NH₄⁺ |

**Tabelle 4: Salze der Verbindungen der Formel (I)**

| | | | | | |
|---|---|---|---|---|---|
| 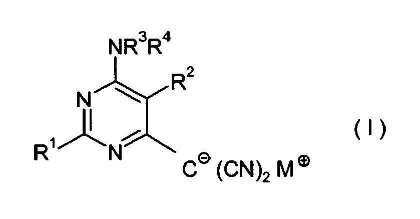 | | | | | |

| Bsp. Nr. | R¹ | R² | R³ | R⁴ | M^{⊕} |
|---|---|---|---|---|---|
| 369. | 1-Cl-cPr | F | H | H | NH₄⁺ |
| 370. | 1-Cl-cPr | Cl | H | H | Li⁺ |
| 371. | 1-Cl-cPr | Cl | H | H | Na⁺ |
| 372. | 1-Cl-cPr | Cl | H | H | K⁺ |
| 373. | 1-Cl-cPr | Cl | H | H | Ca²⁺ |
| 374. | 1-Cl-cPr | Cl | H | H | Mg²⁺ |
| 375. | 1-Cl-cPr | Cl | H | H | NH₄⁺ |
| 376. | 1-Cl-cPr | Cl | H | H | NH₃Me⁺ |
| 377. | 1-Cl-cPr | Cl | H | H | NH₂Me₂⁺ |
| 378. | 1-Cl-cPr | Cl | H | H | NHMe₃⁺ |
| 379. | 1-Cl-cPr | Cl | H | H | NMe₄⁺ |
| 380. | 1-Cl-cPr | Br | H | H | Na⁺ |
| 381. | 1-Cl-cPr | Br | H | H | NH₄⁺ |
| 382. | 1-Cl-cPr | Cl | H | Me | NH₄⁺ |
| 383. | 1-Cl-cPr | Br | H | Me | NH₄⁺ |
| 384. | 2-cPr-Vin | Cl | H | H | NH₄⁺ |
| 385. | 2-di-Me-cPr | Cl | H | H | Li⁺ |
| 386. | 2-di-Me-cPr | Cl | H | H | Na⁺ |
| 387. | 2-di-Me-cPr | Cl | H | H | K⁺ |
| 388. | 2-di-Me-cPr | Cl | H | H | Ca²⁺ |
| 389. | 2-di-Me-cPr | Cl | H | H | Mg²⁺ |
| 390. | 2-di-Me-cPr | Cl | H | H | NH₄⁺ |
| 391. | 2-di-Me-cPr | Cl | H | H | NH₃Me⁺ |
| 392. | 2-di-Me-cPr | Cl | H | H | NH₂Me₂⁺ |
| 393. | 2-di-Me-cPr | Cl | H | H | NHMe₃⁺ |
| 394. | 2-di-Me-cPr | Cl | H | H | NMe₄⁺ |
| 395. | 2-di-Me-cPr | Br | H | H | NH₄⁺ |
| 396. | 2-Me-Vin | Cl | H | H | NH₄⁺ |
| 397. | 2-Ph-Vin | Cl | H | H | NH₄⁺ |
| 398. | 4-Cl-PhCH₂ | Cl | H | H | Na⁺ |
| 399. | 4-Cl-PhCH₂ | Cl | H | H | NH₄⁺ |
| 400. | cBu | Cl | H | H | Na⁺ |
| 401. | cBu | Cl | H | H | NH₄⁺ |
| 402. | cBu | Br | H | H | Na⁺ |
| 403. | cBu | Br | H | H | NH₄⁺ |
| 404. | CF₃ | Cl | H | H | NH₄⁺ |
| 405. | cHex | Cl | H | H | NH₄⁺ |
| 406. | cPr | F | H | H | NH₄⁺ |
| 407. | cPr | Cl | H | H | Li⁺ |
| 408. | cPr | Cl | H | H | Na⁺ |
| 409. | cPr | Cl | H | H | K⁺ |
| 410. | cPr | Cl | H | H | Ca²⁺ |
| 411. | cPr | Cl | H | H | Mg²⁺ |
| 412. | cPr | Cl | H | H | NH₄⁺ |
| 413. | cPr | Cl | H | H | NH₃Me⁺ |
| 414. | cPr | Cl | H | H | NH₂Me₂⁺ |
| 415. | cPr | Cl | H | H | NHMe₃⁺ |
| 416. | cPr | Cl | H | H | NMe₄⁺ |
| 417. | cPr | Br | H | H | Na⁺ |
| 418. | cPr | Br | H | H | NH₄⁺ |
| 419. | cPr | Cl | H | Ac | Na⁺ |
| 420. | cPr | Cl | H | Ac | NH₄⁺ |
| 421. | cPr | Cl | H | Me | NH₄⁺ |
| 422. | cPr | Cl | Me | Me | NH₄⁺ |
| 423. | Hex | Cl | H | H | NH₄⁺ |
| 424. | iPr | Cl | H | H | Na⁺ |
| 425. | iPr | Cl | H | H | NH₄⁺ |
| 426. | iPr | Br | H | H | NH₄⁺ |
| 427. | Me | Cl | H | H | NH₄⁺ |
| 428. | PhCH₂ | Cl | H | H | NH₄⁺ |
| 429. | PhCH₂ | Cl | H | H | Na⁺ |
| 430. | tBu | Cl | H | H | NH₄⁺ |
| 431. | tBu | Br | H | H | NH₄⁺ |
| 432. | Vin | Cl | H | H | Na⁺ |
| 433. | Vin | Cl | H | H | NH₄⁺ |
| 434. | Vin | Br | H | H | NH₄⁺ |

**Physikalische Daten ausgewählter Verbindungen der Tabellen 1-4:**

| Bsp. Nr. | Daten |
|---|---|
| 1. | 7.99 (m, 2H), 7.62 (m, 3H), 7.40 (bs, 2H) |
| 29. | 8.00 (d, 2H), 7.70 (d, 2H), 7.40 (bs, 2H) |
| 41. | 8.08 (d, 2H), 7.60 (d, 2H), 4.40 (bs, 2H), 2.98 (s, 3H) |
| 80. | 7.85 (m, 1H), 7.78 (m, 1H), 7.64 (m, 1H), 7.49 (m, 1H), 7.31 (bs, 2H) |
| 87. | 8.49 (s, 1H), 8.30 (d, 1H), 7.93 (d, 1H), 7.15 (bs, 2H) |
| 110. | 7.83 (t, 1H), 7.71 (d, 1H), 7.70 (bs, 2H), 7.51 (d, 1H) |
| 174. | 7.85 (bs, 2H), 7.61 (m, 3H) |
| 225. | 4.94 (bs, 2H) |
| 238. | 12.40 (bs, 1H), 7.50 (bs, 2H), 2.12 (bs, 1H), 1.01 (m, 4H) |
| 261. | 7.66 (bs, 2H), 2.31 (s, 3H) |
| 266. | 7.30 (bs, 2H), 1.28 (s, 9H) |
| 277. | 8.08 (m, 2H), 7.55 (m, 3H), 7.23 - 6.92 (m, 6H) |
| 293. | 8.21 (d, 2H), 7.48 (d, 2H), 6.36 (bs, 2H) |
| 297. | 8.19 (d, 2H), 7.49 (d, 2H), 7.25 - 6.91 (m, 4H), 6.50 (bs, 2H) |
| 321. | 8.02 (m, 1H), 7.89 (m, 1H), 7.48 (m, 1H), 7.27 (m, 1H), 7.22 - 6.93 (m, 4H), 6.53 (bs, 2H) |
| 324. | 8.61 (s, 1H), 8.42 (d, 1H), 7.82 (d, 1H), 7.22 - 6.92 (m, 4H), 6.59 (bs, 2H) |
| 348. | 7.49 (m, 2H), 7.32 (bs, 2H), 7.19 - 6.91 (m, 4H), 3.91 (s, 3H) |
| 360. | 7.49 (m, 2H), 7.42 (m, 1H), 7.22 - 6.92 (m, 4H), 6.57 (bs, 2H) |

### Methode: ¹H-NMR (Bruker DRX-400, 400 MHz, 294K, DMSO-d₆, TMS = 0.0 ppm)

**Tabelle 5: Verbindungen der Formel (II)**

| | | | |
|---|---|---|---|
| 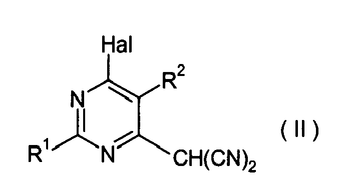 | | | |

| Bsp. Nr. | R¹ | R² | Hal |
|---|---|---|---|
| 435. | Ph | Cl | F |
| 436. | Ph | Br | F |
| 437. | Ph | Cl | Cl |
| 438. | Ph | Br | Cl |
| 439. | Ph | Cl | Br |
| 440. | Ph | Br | Br |
| 441. | 4-OMe-Ph | Cl | Cl |
| 442. | 4-OMe-Ph | Br | Cl |
| 443. | 4-NO₂-Ph | Cl | Cl |
| 444. | 4-NO₂-Ph | Br | Cl |
| 445. | 4-Me-Ph | Cl | Cl |
| 446. | 4-Me-Ph | Br | Cl |
| 447. | 4-I-Ph | Cl | Cl |
| 448. | 4-I-Ph | Br | Cl |
| 449. | 4-F-Ph | Cl | Cl |
| 450. | 4-F-Ph | Br | Cl |
| 451. | 4-Cl-Ph | Cl | Cl |
| 452. | 4-Cl-Ph | Br | Cl |
| 453. | 4-CF₃-Ph | Cl | Cl |
| 454. | 4-CF₃-Ph | Br | Cl |
| 455. | 4-Br-Ph | Cl | Cl |
| 456. | 4-Br-Ph | Br | Cl |
| 457. | 3-OMe-4-F-Ph | Cl | Cl |
| 458. | 3-OMe-4-F-Ph | Br | Cl |
| 459. | 3-Me-Ph | Cl | Cl |
| 460. | 3-Me-Ph | Br | Cl |
| 461. | 3-Me-4-Cl-Ph | Cl | Cl |
| 462. | 3-Me-4-Cl-Ph | Br | Cl |
| 463. | 3-F-Ph | Cl | Cl |
| 464. | 3-F-Ph | Br | Cl |
| 465. | 3-Cl-Ph | Cl | Cl |
| 466. | 3-Cl-Ph | Br | Cl |
| 467. | 3-CF₃-Ph | Cl | Cl |
| 468. | 3-CF₃-Ph | Br | Cl |
| 469. | 3-CF₃-4-Cl-Ph | Cl | Cl |
| 470. | 3-CF₃-4-Cl-Ph | Br | Cl |
| 471. | 3,6-di-Cl-Ph | Cl | Cl |
| 472. | 3,6-di-Cl-Ph | Br | Cl |
| 473. | 3,5-di-Cl-Ph | Cl | Cl |
| 474. | 3,5-di-Cl-Ph | Br | Cl |
| 475. | 3,5-di-CF₃-Ph | Cl | Cl |
| 476. | 3,5-di-CF₃-Ph | Br | Cl |
| 477. | 3,4-di-OMe-Ph | Cl | Cl |
| 478. | 3,4-di-OMe-Ph | Br | Cl |
| 479. | 3,4-di-Cl-Ph | Cl | Cl |
| 480. | 3,4-di-Cl-Ph | Br | Cl |
| 481. | 2-F-6-Cl-Ph | Cl | Cl |
| 482. | 2-F-6-Cl-Ph | Br | Cl |
| 483. | 2-F-4-Cl-Ph | Cl | Cl |
| 484. | 2-F-4-Cl-Ph | Br | Cl |
| 485. | 2-F-4-Cl-5-OMe-Ph | Cl | Cl |
| 486. | 2-F-4-Cl-5-OMe-Ph | Br | Cl |
| 487. | 2-F-4,5-di-Cl-Ph | Cl | Cl |
| 488. | 2-F-4,5-di-Cl-Ph | Br | Cl |
| 489. | 2-F-3-SMe-4-Cl-Ph | Cl | Cl |
| 490. | 2-F-3-SMe-4-Cl-Ph | Br | Cl |
| 491. | 2-F-3-OMe-4-Cl-Ph | Cl | Cl |
| 492. | 2-F-3-OMe-4-Cl-Ph | Br | Cl |
| 493. | 2-F-3-OEt-4-Cl-Ph | Cl | Cl |
| 494. | 2-F-3-OEt-4-Cl-Ph | Br | Cl |
| 495. | 2-F-3-OCF₃-4-Cl-Ph | Cl | Cl |
| 496. | 2-F-3-OCF₃-4-Cl-Ph | Br | Cl |
| 497. | 2-F-3-NMe₂-4-Cl-Ph | Cl | Cl |
| 498. | 2-F-3-NMe₂-4-Cl-Ph | Br | Cl |
| 499. | 2-F-3-Me-4-Cl-Ph | Cl | Cl |
| 500. | 2-F-3-Me-4-Cl-Ph | Br | Cl |
| 501. | 2-Cl-Ph | Cl | Cl |
| 502. | 2-Cl-Ph | Br | Cl |
| 503. | 2,6-di-F-3-OMe-4-Cl-Ph | Cl | Cl |
| 504. | 2,6-di-F-3-OMe-4-Cl-Ph | Br | Cl |
| 505. | 2,6-di-Cl-Ph | Cl | Cl |
| 506. | 2,6-di-Cl-Ph | Br | Cl |
| 507. | 2,5-di-Cl-Ph | Cl | Cl |
| 508. | 2,5-di-Cl-Ph | Br | Cl |
| 509. | 2,4-di-F-5-OMe-Ph | Cl | Cl |
| 510. | 2,4-di-F-5-OMe-Ph | Br | Cl |
| 511. | 2,4-di-F-3-OMe-Ph | Cl | Cl |
| 512. | 2,4-di-F-3-OMe-Ph | Br | Cl |
| 513. | 2,4-di-Cl-Ph | Cl | Cl |
| 514. | 2,4-di-Cl-Ph | Br | Cl |
| 515. | 2,4-di-Cl-5-F-Ph | Cl | Cl |
| 516. | 2,4-di-Cl-5-F-Ph | Br | Cl |
| 517. | 2,4-di-Cl-3-OMe-Ph | Cl | Cl |
| 518. | 2,4-di-Cl-3-OMe-Ph | Br | Cl |
| 519. | 2,4,6-tri-Cl-Ph | Cl | Cl |
| 520. | 2,4,6-tri-Cl-Ph | Br | Cl |
| 521. | 1-Cl-cPr | Cl | Cl |
| 522. | 1-Cl-cPr | Br | Cl |
| 523. | 2-cPr-Vin | Cl | Cl |
| 524. | 2-cPr-Vin | Br | Cl |
| 525. | 2-di-Me-cPr | Cl | Cl |
| 526. | 2-di-Me-cPr | Br | Cl |
| 527. | 2-Me-Vin | Cl | Cl |
| 528. | 2-Me-Vin | Br | Cl |
| 529. | 2-Ph-Vin | Cl | Cl |
| 530. | 2-Ph-Vin | Br | Cl |
| 531. | 4-Cl-PhCH₂ | Cl | Cl |
| 532. | 4-Cl-PhCH₂ | Br | Cl |
| 533. | cBu | Cl | Cl |
| 534. | cBu | Br | Cl |
| 535. | CF₃ | Cl | Cl |
| 536. | CF₃ | Br | Cl |
| 537. | cHex | Cl | Cl |
| 538. | cHex | Br | Cl |
| 539. | cPr | Cl | Cl |
| 540. | cPr | Br | Cl |
| 541. | Hex | Cl | Cl |
| 542. | Hex | Br | Cl |
| 543. | iPr | Cl | Cl |
| 544. | iPr | Br | Cl |
| 545. | Me | Cl | F |
| 546. | Me | Br | F |
| 547. | Me | Cl | Cl |
| 548. | Me | Br | Cl |
| 549. | Me | Cl | Br |
| 550. | Me | Br | Br |
| 551. | PhCH₂ | Cl | Cl |
| 552. | PhCH₂ | Br | Cl |
| 553. | tBu | Cl | Cl |
| 554. | tBu | Br | Cl |
| 555. | Vin | Cl | Cl |
| 556. | Vin | Br | Cl |

**Physikalische Daten ausgewählter Verbindungen der Tabelle 5:**

| Bsp. Nr. | Daten |
|---|---|
| 451. | 8.21 (d, 2H), 7.54 (d, 2H) |
| 463. | 8.19 - 8.14 (m, 2H), 7.59 - 7.48 (m, 2H) |
| 505. | 7.58 - 7.53 (d, 2H), 7.50 - 7.45 (m, 1H) |
| 547. | 2.28 (s, 3H) |

### Methode: ¹H-NMR (Bruker DRX-400, 400 MHz, 294K, DMSO-d₆, TMS = 0.0 ppm)

### B. Formulierungsbeispiele

a) Ein Stäubemittel wird erhalten, indem man 10 Gew.-Teile einer erfindungsgemäßen Verbindung und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer erfindungsgemäßen Verbindung, 64 Gew.-Teile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gew.-Teile einer erfindungsgemäßen Verbindung mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277 °C) mischt und in einer Reibkugelmühle auf eine Feinheit von unter 5 Mikron vermahlt.
d) Ein emulgierbares Konzentrat wird erhalten aus 15 Gew.-Teilen einer erfindungsgemäßen Verbindung, 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man
   75 Gew.-Teile einer erfindungsgemäßen Verbindung,
   10 Gew.-Teile ligninsulfonsaures Calcium,
   5 Gew.-Teile Natriumlaurylsulfat,
   3 Gew.-Teile Polyvinylalkohol und
   7 Gew.-Teile Kaolin
   mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man
   25 Gew.-Teile einer erfindungsgemäßen Verbindung
   5 Gew.-Teile 2,2'-dinaphthylmethan-6,6'-disulfonsaures Natrium
   2 Gew.-Teile oleoylmethyltaurinsaures Natrium,
   1 Gew.-Teil Polyvinylalkohol,
   17 Gew.-Teile Calciumcarbonat und
   50 Gew.-Teile Wasser
   auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

Herbizide Wirkung bzw. Kulturpflanzenverträglichkeit im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt. Die in Form von benetzbaren Pulvern (WP) oder als Emulsionskonzentrate (EC) formulierten erfindungsgemäßen Verbindungen werden dann als wässrige Suspension bzw. Emulsion mit einer Wasseraufwandmenge von umgerechnet 600 bis 800 I/ha unter Zusatz von 0,2 Gew.-% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen).

Wie die Ergebnisse zeigen, weisen erfindungsgemäße Verbindungen eine gute herbizide Nachauflaufwirksamkeit gegen ein breites Spektrum von Ungräsern und Unkräutern auf. Beispielsweise hat die Verbindung Nr. 1 und andere Verbindungen aus den Tabellen 1 bis 4 sehr gute herbizide Wirkung von mindestens 80 % gegen Schadpflanzen wie Matricaria inodora, Polygonum (Fallopia) convolvulus und Veronica persica im Nachauflaufverfahren bei einer Aufwandmenge von 0.32 kg und weniger Aktivsubstanz pro Hektar. Gleichzeitig lassen erfindungsgemäße Verbindungen Gramineen-Kulturen wie Gerste, Weizen, Roggen, Hirse, Mais oder Reis im Nachauflaufverfahren selbst bei hohen Wirkstoffdosierungen ungeschädigt. Einige Substanzen schonen darüber hinaus auch zweikeimblättrige Kulturen wie Soja, Baumwolle, Raps, Zuckerrüben oder Kartoffeln. Die erfindungsgemäßen Verbindungen zeigen teilweise eine hohe Selektivität und eignen sich deshalb im Nachauflaufverfahren zur Bekämpfung von unerwünschtem Pflanzenwuchs in landwirtschaftlichen Kulturen.

## Patentansprüche

1. Verbindung der Formel (1) und deren N-Oxide und agrochemisch geeignete
Salze, 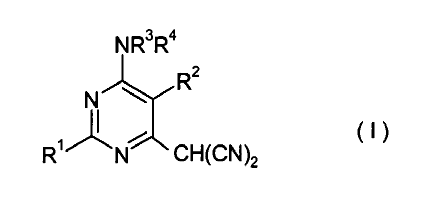 worin die Reste folgende Bedeutung aufweisen:
R¹ ist Phenyl, optional substituiert mit 1-3 Resten R^{x}, welche unabhängig
voneinander bedeuten Halogen, Cyano, Nitro, (C₁-C₆)Alkyl, (C₁-C₆)Haloalkyl, (C₁-C₄)Hydroxyalkyl, (C₂-C₄)Alkoxyalkyl, (C₂-C₄)Haloalkoxyalkyl, (C₂-C₄)Alkenyl, (C₂-C₄)Haloalkenyl, (C₂-C₄)Alkinyl, (C₃-C₄)Haloalkinyl, Hydroxy, (C₁-C₄)Alkoxy, (C₁-C₄)Haloalkoxy, (C₂-C₄)Alkenyloxy, (C₂-C₄)Haloalkenyloxy, (C₂-C₄)Alkinyloxy, (C₃-C₄)Haloalkinyloxy, (C₁-C₄)Alkylthio, (C₁-C₄)Haloalkylthio, (C₁-C₄)Alkylsulfinyl, (C₁-C₄)Haloalkylsulfinyl, (C₁-C₄)Alkylsulfonyl, (C₁-C₄)Haloalkylsulfonyl, (C₂-C₄)Alkenylthio, (C₂-C₄)Haloalkenylthio, (C₂-C₄)Alkenylsulfinyl, (C₂-C₄)Haloalkenylsulfinyl, (C₂-C₄)Alkenylsulfonyl, (C₂-C₄)Haloalkenylsulfonyl, (C₂-C₄)Alkinylthio, (C₃-C₄)Haloalkinylthio, (C₃-C₄)Alkinylsulfinyl, (C₃-C₄)Haloalkinylsulfinyl, (C₃-C₄)Alkinylsulfonyl, (C₃-C₄)Haloalkinylsulfonyl, (C₁-C₆)Alkylamino, (C₂-C₈)Dialkylamino, (C₂-C₆)Alkylcarbonyl, (C₂-C₆)Alkoxycarbonyl, (C₂-C₆)Alkylaminocarbonyl, (C₃-C₈)Dialkylaminocarbonyl, (C₃-C₆)Trialkylsilyl, Phenyl, Phenoxy oder einen 5- oder 6-gliedrigen heteroaromatischen Ring, jeder Phenyl-Ring, Phenoxy-Ring oder 5-oder 6-gliedrige heteroaromatische Ring optional substituiert mit 1-3
Substituenten unabhängig voneinander ausgewählt aus R²⁸; oder zwei benachbarte Reste R^{x} bilden gemeinsam eine Gruppe
-OCH₂O-, -CH₂CH₂O-, -OCH₂CH₂O-, -OCH(CH₃)O-, -OC(CH₃)₂O-, -OCF₂O-,
-CF₂CF₂O-, -OCF₂CF₂O- oder -CH=CH-CH=CH-; oder
(C₁-C₆)-Alkyl, optional substituiert mit 1-3 Resten R^{y}, welche unabhängig voneinander Halogen, (C₂-C₆)Alkenyl, (C₂-C₆)Haloalkenyl, (C₁-C₃)Alkoxy, (C₁-C₂)Haloalkoxy, (C₁-C₃)Alkylthio oder (C₁-C₂)Haloalkylthio bedeuten; oder
(C₂-C₆)-Alkenyl, optional substituiert mit 1-3 Resten R², welche unabhängig voneinander Halogen, (C₁-C₆)Alkyl, (C₁-C₆)Haloalkyl, (C₁-C₃)Alkoxy, (C₁-C₂)Haloalkoxy, (C₁-C₃)Alkylthio oder (C₁-C₂)Haloalkylthio bedeuten;
R² ist H, F, Cl, Br, I, CN, NO₂, OR⁵, SR⁶ oder N(R⁷)R⁸; worin R⁵ H, (C₁-C₄)Alkyl
oder (C₁-C₃)Haloalkyl ist, R⁶ H, (C₁-C₄)Alkyl oder (C₁-C₃)Haloalkyl ist und R⁷ und R⁸ sind unabhängig H oder (C₁-C₄)Alkyl;
R³ ist H, (C₁-C₄)Alkyl optional substituiert mit 1-2 Resten R⁹, (C₂-C₄)Alkenyl
optional substituiert mit 1-2 Resten R¹⁰, oder (C₂-C₄)Alkinyl optional substituiert mit 1-2 Resten R¹¹; oder R³ ist C(=O)R¹², NO₂, OR¹³, S(O)₂R¹⁴, N(R¹⁵)R₁₆ oder N=C(R¹⁷)R¹⁸;
R⁴ ist H, (C₁-C₄)Alkyl optional substituiert mit Resten 1-2 R⁹, oder C(=O)R¹²; oder
R³ und R⁴ bilden gemeinsam eine Gruppe -(CH₂)₄-, -(CH₂)₅-, -CH₂CH=CHCH₂- oder
-(CH₂)₂O(CH₂)₂-, jede Gruppe optional substituiert mit 1-2 Resten R¹⁹; oder
R³ und R⁴ bilden gemeinsam eine Gruppe =C(R²⁰)N(R²¹)R²² oder =C(R²³)OR²⁴;
dabei ist jeder Rest R⁹, R¹⁰ und R¹¹ unabhängig voneinander Halogen, (C₁-C₃)Alkoxy, (C₁-C₃)Haloalkoxy, (C₁-C₃)Alkylthio, (C₁-C₃)Haloalkylthio, Amino, (C₁-C₃)Alkylamino, (C₂-C₄)Dialkylamino oder (C₂-C₄)Alkoxycarbonyl;
R¹² ist unabhängig voneinander H, (C₁-C₄)Alkyl, (C₁-C₃)Haloalkyl, (C₁-C₄)Alkoxy, Phenyl, Phenoxy oder Benzyloxy;
R¹³ ist H, (C₁-C₄)Alkyl, (C₁-C₃)Haloalkyl oder CHR²⁵C(O)OR²⁶;
R¹⁴ ist (C₁-C₄)Alkyl oder (C₁-C₃)Haloalkyl;
R¹⁵ ist H, (C₁-C₄)Alkyl oder C(=O)R²⁷;
R¹⁶ ist H oder (C₁-C₄)Alkyl;
R¹⁷ ist H, (C₁-C₄)Alkyl oder Phenyl optional substituiert mit 1-3 Resten, welche voneinander unabhängig CH₃, Cl oder OCH₃ bedeuten;
R¹⁸ ist H oder (C₁-C₄)Alkyl; oder R¹⁷ und R¹⁸ bilden gemeinsam eine Gruppe -(CH₂)₄- oder -(CH₂)₅-;
R¹⁹ ist unabhängig voneinander Halogen, (C₁-C₃)Alkyl, (C₁-C₃)Alkoxy, (C₁-C₃)Haloalkoxy, (C₁-C₃)Alkylthio, (C₁-C₃)Haloalkylthlo, Amino, (C₁-C₃)Alkylamino, (C₂-C₄)Dialkylamino oder (C₂-C₄)Alkoxycarbonyl;
R²⁰ ist H oder (C₁-C₄)Alkyl;
R²¹ und R²² sind unabhängig voneinander H oder (C₁-C₄)Alkyl; oder R²¹ und R²² bilden gemeinsam eine Gruppe -(CH₂)₄-, -(CH₂)₅-, -CH₂CH=CHCH₂- oder -(CH₂)₂O(CH₂)₂-;
R²³ ist H oder (C₁-C₄)Alkyl;
R²⁴ ist (C₁-C₄)Alkyl;
R²⁵ ist H, (C₁-C₄)Alkyl, (C₁-C₃)Haloalkyl, (C₁-C₄)Alkoxy, Phenyl, Phenoxy oder Benzyloxy;
R²⁶ ist H, (C₁-C₄)Alkyl oder (C₁-C₄)Alkoxy;
R²⁷ ist H, C₁-C₄ Alkyl oder Benzyl; und
R²⁸ ist unabhängig voneinander Halogen, Cyano, Nitro, (C₁-C₆)Alkyl, (C₁-C₆)Haloalkyl, (C₂-C₄)Alkenyl, (C₂-C₄)Haloalkenyl, (C₃-C₄)Alkinyl, (C₃-C₄)Haloalkinyl, (C₁-C₄)Alkoxy, (C₁-C₄)Haloalkoxy, (C₁-C₄)Alkylthio, (C₁-C₄)Haloalkylthio, (C₁-C₄)Alkylsulfinyl, (C₁-C₄)Alkylsulfonyl, (C₁-C₄)Alkylamino, (C₂-C₈)Dialkylamino, (C₂-C₄)Alkylcarbonyl, (C₂-C₆)Alkoxycarbonyl, (C₂-C₆)Alkylaminocarbonyl, (C₃-C₈)Dialkylaminocarbonyl oder (C₃-C₆)Trialkylsilyl.

2. Verbindung der Formel (I) und deren N-Oxide und agrochemisch geeignete Salze gemäß Anspruch 1, worin die Reste folgende Bedeutung aufweisen:
R¹ ist Phenyl, optional substituiert mit 1-3 Resten R^{x}, welche unabhängig voneinander bedeuten Halogen, Cyano, Nitro, (C₁-C₆)Alkyl, (C₁-C₆)Haloalkyl, (C₂-C₄)Alkoxyalkyl, (C₂-C₄)Haloalkoxyalkyl, (C₂-C₄)Alkenyl, (C₂-C₄)Haloalkenyl, (C₂-C₄)Alkinyl, (C₃-C₄)Haloalkinyl, Hydroxy, (C₁-C₄)Alkoxy, (C₁-C₄)Haloalkoxy, (C₁-C₄)Alkylthio, (C₁-C₄)Haloalkylthio, (C₁-C₄)Alkylsulfinyl, (C₁-C₄)Alkylsulfonyl, (C₁-C₆)Alkylamino, (C₂-C₈)Dialkylamino, -OCH₂O-, -CH₂CH₂O-, -OCH₂CH₂O-, -OCH(CH₃)O-, -OC(CH₃)₂O-, -OCF₂O-, -CF₂CF₂O- oder -OCF₂CF₂O-; oder
(C₁-C₆)Alkyl, optional substituiert mit 1-3 Resten R^{y}, welche unabhängig voneinander bedeuten Halogen, (C₂-C₆)Alkenyl, (C₂-C₆)Haloalkenyl, (C₁-C₃)Alkoxy, (C₁-C₂)Haloalkoxy, (C₁-C₃)Alkylthio oder (C₁-C₂)Haloalkylthio; oder
(C₂-C₆)Alkenyl, optional substituiert mit 1-3 Resten R^{z}, welche unabhängig voneinander bedeuten Halogen, (C₁-C₆)Alkyl, (C₁-C₆)Haloalkyl, (C₁-C₃)Alkoxy, (C₁-C₂)Haloalkoxy, (C₁-C₃)Alkylthio oder (C₁-C₂)Haloalkylthio;
R² ist H, F, Cl, Br, I, CN oder NO₂;
R³ ist H, (C₁-C₄)Alkyl, (C₂-C₄)Alkenyl, (C₂-C₄)Alkinyl, C(=O)R¹², OR¹³, N(R¹⁵)R¹⁶
oder N=C(R¹⁷)R¹⁸;
R⁴ ist H oder (C₁-C₄)Alkyl, optional substituiert mit 1-2 Resten R⁹, oder C(=O)R¹²;
oder
R³ und R⁴ bilden gemeinsam eine Gruppe -(CH₂)₄-, -(CH₂)₅-, -(CH₂)₂O(CH₂)₂- oder
=C(R²⁰)N(R²¹)R²²;
dabei ist jeder Rest R⁹, R¹⁰ und R¹¹ unabhängig voneinander Halogen, (C₁-C₃)Alkoxy, (C₁-C₃)Haloalkoxy, (C₁-C₃)Alkylthio, (C₁-C₃)Haloalkylthio, Amino, (C₁-C₃)Alkylamino, (C₂-C₄)Dialkylamino oder (C₂-C₄)Alkoxycarbonyl;
R¹² ist H, (C₁-C₄)Alkyl, (C₁-C₃)Haloalkyl, (C₁-C₄)Alkoxy, Phenyl, Phenoxy oder Benzyloxy;
R¹³ ist H, (C₁-C₄)Alkyl, (C₁-C₃)Haloalkyl oder CHR²⁵C(O)OR²⁶;
R¹⁵ ist H, (C₁-C₄)Alkyl oder C(=O)R²⁷;
R¹⁶ ist H oder (C₁-C₄)Alkyl;
R¹⁷ ist H, (C₁-C₄)Alkyl oder Phenyl optional substituiert mit 1-3 Resten, welche voneinander unabhängig CH₃, Cl oder OCH₃ bedeuten;
R¹⁸ ist H oder (C₁-C₄)Alkyl; oder R¹⁷ und R¹⁸ bilden gemeinsam eine Gruppe -(CH₂)₄- oder -(CH₂)₅-;
R²⁰ ist H oder (C₁-C₄)Alkyl;
R²¹ und R²² sind unabhängig voneinander H oder (C₁-C₄)Alkyl; oder R²¹ and R²² bilden gemeinsam eine Gruppe -(CH₂)₄-, -(CH₂)₅-, -CH₂CH=CHCH₂- oder
-(CH₂)₂O(CH₂)₂-;
R²⁵ ist H, (C₁-C₄)Alkyl, (C₁-C₃)Haloalkyl, (C₁-C₄)Alkoxy, Phenyl, Phenoxy oder Benzyloxy; und
R²⁶ ist H, (C₁-C₄)Alkyl oder (C₁-C₄)Alkoxy.

3. Verbindung der Formel (I) und deren N-Oxide und agrochemisch geeignete Salze gemäß Anspruch 1 oder 2, worin die Reste folgende Bedeutung aufweisen:
R¹ ist Phenyl, optional substituiert mit 1-3 Resten R^{x}, welche unabhängig voneinander bedeuten Halogen, Cyano, Nitro, (C₁-C₆)Alkyl, (C₁-C₆)Haloalkyl, (C₁-C₄)Alkoxy, (C₁-C₄)Haloalkoxy, (C₁-C₆)Alkylamino, (C₂-C₈)Dialkylamino,
-OCH₂O- -OCH₂CH₂O- oder -OCH(CH₃)O-; oder
(C₁-C₆)Alkyl, optional substituiert mit 1-3 Resten R^{y}, welche unabhängig voneinander bedeuten Halogen, (C₁-C₃)Alkoxy oder (C₁-C₂)Haloalkoxy; oder
(C₂-C₆)Alkenyl, optional substituiert mit 1-3 Resten R^{z}, welche unabhängig voneinander bedeuten Halogen, (C₁-C₆)Alkyl, (C₁-C₆)Haloalkyl, (C₁-C₃)Alkoxy oder (C₁-C₂)Haloalkoxy;
R² ist F, Cl, Br oder I;
R³ ist H, (C₁-C₄)Alkyl, (C₂-C₄)Alkenyl, C(=O)R¹², worin R¹² H, (C₁-C₄)Alkyl, (C₁-C₃)Haloalkyl, (C₁-C₄)Alkoxy, Phenyl, Phenoxy oder Benzyloxy ist,
oder OR¹³, worin R¹³ H, (C₁-C₄)Alkyl, (C₁-C₃)Haloalkyl oder CHR²⁵C(O)OR²⁶ ist, worin R²⁵ H, (C₁-C₄)Alkyl, (C₁-C₃)Haloalkyl, (C₁-C₄)Alkoxy, Phenyl, Phenoxy oder Benzyloxy ist, und R²⁶ H, (C₁-C₄)Alkyl oder (C₁-C₄)Alkoxy ist;
R⁴ ist H oder (C₁-C₄)Alkyl; oder
R³ und R⁴ bilden gemeinsam eine Gruppe -(CH₂)₂O(CH₂)₂-.

4. Agrochemisches Mittel, enthaltend a) mindestens eine Verbindung der Formel (I) oder deren N-Oxide und agrochemisch geeignete Salze, wie in einem oder mehreren der Ansprüche 1 bis 3 definiert, und b) im Pflanzenschutz übliche Hilfsund Zusatzstoffe.

5. Agrochemisches Mittel, enthaltend a) mindestens eine Verbindung der Formel (I) oder deren N-Oxide und agrochemisch geeignete Salze, wie in einem oder mehreren der Ansprüche 1 bis 3 definiert, b) einen oder mehrere von Komponente a) verschiedene agrochemische Wirkstoffe, und optional c) im Pflanzenschutz übliche Hilfs- und Zusatzstoffe.

6. Verfahren zur Bekämpfung von unerwünschten Pflanzen oder zur Wachstumsregulierung von Pflanzen, wobei eine wirksame Menge mindestens einer Verbindung der Formel (I) oder deren N-Oxide und agrochemisch geeignete Salze, wie in einem oder mehreren der Ansprüche 1 bis 3 definiert, auf die Pflanzen, das Saatgut oder die Fläche, auf der die Pflanzen wachsen, appliziert wird.

7. Verwendung von Verbindungen der Formel (I) oder deren N-Oxide und agrochemisch geeignete Salze, wie in einem oder mehreren der Ansprüche 1 bis 3 definiert, als Herbizide oder Pflanzenwachstumsregulatoren.

8. Verwendung nach Anspruch 7, wobei die Verbindungen der Formel (I) oder deren N-Oxide und agrochemisch geeignete Salze zur Bekämpfung von Schadpflanzen oder zur Wachstumsregulierung in Pflanzenkulturen eingesetzt werden.

9. Verwendung nach Anspruch 8, wobei die Kulturpflanzen transgene oder nicht transgene Kulturpflanzen sind.

10. Verfahren zur Herstellung einer Verbindung der Formel (I) oder deren N-Oxide und agrochemisch geeignete Salze, wie in einem oder mehreren der Ansprüche 1 bis 3 definiert, wobei man a) eine Halogenverbindung der Formel (II) 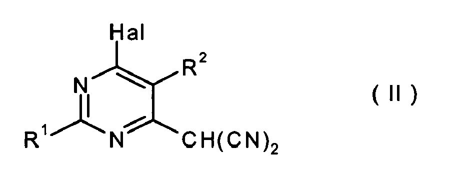 mit einem Amin der Formel (III) HNR³R⁴ umsetzt, wobei Hal ein Halogenatom ist, die Reste R¹, R² in Formel (II) und R³, R⁴ in Formel (III) wie in Formel (I) in einem oder mehreren der Ansprüche 1 bis 3 definiert sind, und b) gegebenenfalls die in Schritt a) enthaltene Verbindung der Formel (I) nach üblichen Methoden in ein N-Oxid oder ein agrochemisch geeignetes Salz überführt.

11. Verbindung der Formel (II), worin die Reste R¹ und R² wie in Formel (I) in einem oder mehreren der Ansprüche 1 bis 3 definiert sind.
